# EUROPEAN PATENT APPLICATION

(11) **EP 3 659 438 A1**
(43) Date of publication of application: **03.06.2020**
(21) Application number: 20151019.5
(22) Date of filing: 06.10.2016
(51) Int. Cl.: A01N 43/40, A01N 43/50, A01N 43/54, A01N 43/82, A01N 43/88, A01N 47/06, A01N 55/00

(54) **COMPOUNDS AND COMPOSITIONS HAVING KNOCK-DOWN OR BLOOD FEED INHIBITION ACTIVITY AGAINST INSECT PESTS**

(30) Priority: 06.10.2015 US 201562238050 P
(62) Divisional of application: 16777706.9
(71) Applicant: SYNGENTA PARTICIPATIONS AG, 4058 Basel (CH)
(72) Inventor: HOPPE, Mark, 4332 Stein (CH); HUETER, Ottmar, Franz, 4332 Stein (CH); MAIENFISCH, Peter, 4332 Stein (CH); WEGE, Philip, Bracknell, Berkshire RG42 6EY (GB); PITTERNA, Thomas, 4332 Stein (CH); BOEGER, Manfred, deceased (CH)
(74) Representative: SYNGENTA IP

(57) **Abstract**

With the present invention it has now been found that certain 4-(trifluoromethyl)pyridine compounds and active compound compositions comprising such compounds are suitable for controlling nuisance, disease carrying or haematophagous (blood feeding) insects pests incl. dipteran, triatominae and cimicidae insect pests by knockdown or by or blood feed inhibition. In one embodiment, dipteran pests are selected from flies and mosquitoes, including insecticide-resistant flies and mosquitoes, as well as fly and mosquito vectors of pathogenic disease. Target cimicidae insect pests are selected from bed bugs. Target triatominae pests are selected from kissing bugs Other aspects of the present invention will also be apparent in the detailed description which follows.

## Description

The field of the invention relates to insect pest control and in particular dipteran, cimicidae and triatominae insect control. The active compounds and active compound compositions of this invention are particularly useful to knockdown or inhibit blood feeding of insects such as mosquitos, flies, kissing bugs and bed bugs that are a nuisance, and those which are haematophagous, or are vectors of human or animal diseases and/or cause allergic reactions.

More specifically, the present invention relates to control of nuisance, disease carrying or haematophagous insect pests by knockdown or blood feed inhibition with certain active pyridine compounds and active compound compositions comprising such pyridine compounds, and to related products, methods, treated substrates, and integrated insect pest management solutions.

House flies and stable flies are common dipteran insects around horse barns, stables, and corrals. Persistent house flies are very annoying and potential carriers of human and animal pathogens whereas stable flies give painful bites making activities unpleasant for humans and making horses more difficult to manage. Thus, the effective control of such flies is highly desirable.

Mosquitoes are very harmful dipteran insects particularly in view of hygiene as these insects can be vectors of human pathogenic disease such as dengue, yellow fever, encephalitis, malaria, filariasis, chikungunya, and Zika virus. Mosquito control manages the population of mosquitoes to reduce their damage to human health, economies, and enjoyment. Mosquito-control operations are targeted against three different problems:
1. Nuisance mosquitoes bother people around homes or in parks and recreational areas;
2. Economically important mosquitoes reduce real estate values, adversely affect tourism and related business interests, or negatively impact livestock or poultry production;
3. Public health is the focus when mosquitoes are vectors, or transmitters, of infectious disease.

Bed bugs are parasitic insects of the family Cimicidae. They feed preferentially on human blood and the blood of other warm-blooded animals and are mainly active at night. Bites from bed bugs often go undetected at the time, and in many instances there is no visible sign of the bite. However, they cause a skin condition known as cimicosis which is accompanied by serious skin itching which can lead to anxiety, stress and insomnia, as well as secondary infection as a result of scratching. Largely because of their nocturnal habits, bed bugs typically are hard to detect and eradicate.

Insecticidal compositions have commonly been used to control dipteran insect pests. In order for an insecticide to act at its target site, it must enter the insect through one or more absorption routes, including absorption through the cuticle, through proprioceptive and/or tactile receptors, orally through the consumption of treated foliage, sap or edible bait, or by inhalation through the spiracles as a vapour. Among the characteristics used to evaluate contact insecticidal compositions are the insecticide's 'knockdown' and 'mortality' characteristics. Knockdown refers to a quick, short-term immobilization that can precede mortality of the insect pest. In some cases, insect pests can recover from knockdown immobilization.

Due to natural selection, dipteran insect pests including flies and mosquitoes can develop a resistance to chemicals and therefore there is a continuous need to improve the currently available active compound compositions and methods of use thereof in order to allow for efficient fly and/or mosquito control and resistance management. For example, metabolic resistance confers resistance to certain pyrethroids, whereas target-based resistance extends to all pyrethroids and DDT, and is known as knockdown resistance (kdr).

Pyrethroid resistance, caused either by specific detoxification enzymes or an altered target site mechanism (kdr-type mutations in the sodium channels), has been reported in most continents in the majority of medically important mosquitoes species, such as *Anopheles gambiae* in Africa and *Aedes aegypti* in Asia. If such resistance continues to develop and spread at the current rate, it may render such insecticides ineffective in their current form in the not too distant future. Such a scenario would have potentially devastating consequences in public health terms, since there are as yet no obvious alternatives to many of the uses of pyrethroids.

The pesticide flonicamid and its metabolites TFNA, TFNA-AM, and TFNG are known (see, e.g., US patent no. 5,360,806). Flonicamid was developed in 2000 as a selective agent against aphids and other sucking insects. The mode of action has been identified as suppressing feeding and movement by aphids. While the activity of flonicamid is good against certain insects, it has not been shown to be active against dipteran pests such as flies or mosquitoes, particularly by knockdown or blood feed inhibition. Moreover, no fly or mosquito knockdown or blood feed inhibiting activity of the above-noted flonicamid metabolites have been reported.

With the present invention it has now been found that certain pyridine compounds (compared to similar analogous compounds) and active compound compositions comprising such pyridine compounds are surprisingly useful for controlling nuisance, disease carrying or haematophagous (blood feeding) insects pests incl. dipteran, triatominae and cimicidae insect pests by knockdown or by or blood feed inhibition. In one embodiment, dipteran pests are selected from flies and mosquitoes, including insecticide-resistant flies and mosquitoes, as well as fly and mosquito vectors of pathogenic disease. Target cimicidae insect pests are selected from bed bugs. Target triatominae pests are selected from kissing bugs. Other aspects of the present invention such as usefulness for decreasing dipteran (e.g., mosquito), triatominae or cimicidae insect vector populations will also be apparent in the detailed description which follows.

More specifically, the active compounds suitable for use in the active compound compositions, methods, products, treated substrates, and integrated solutions of the invention are selected from certain 4-(trifluoromethyl)pyridine compounds that are capable of being "picked-up" by target dipteran, triatominae and cimicidae insect pests and cause rapid knockdown of the target insect or inhibit the target insect from taking a blood meal if such insect is haematophagous. In partciular, the inventive 4-(trifluoromethyl)pyridine compounds and related active compound compositions exhibit rapid knockdown or blood feed inhibiting activity against such insect pests without requiring oral administration such as by consumption of treated bait or other foodsource containing such compounds.

More particularly, the present invention provides a method for controlling nuisance, disease carrying or haematophagous dipteran, triatominae and/or cimicidae insect pests by knockdown or by blood feed inhibition with one or more 4-(trifluoromethyl)pyridine compounds respresented by the structural formulae I.1 - 1.29 as shown inTable 1 below.

**TABLE 1**

| | | | | | |
|---|---|---|---|---|---|
| 1.1 | 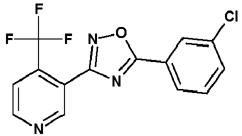 | 1.2 | 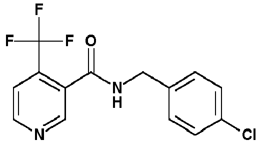 | 1.3 | 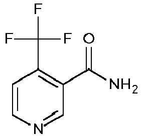 |
| 1.4 | 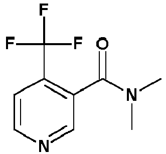 | 1.5 | 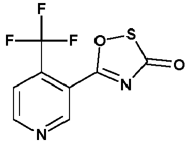 | 1.6 | 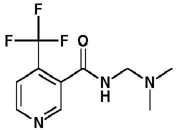 |
| 1.7 | 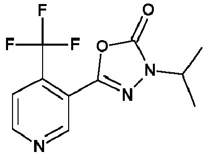 | 1.8 | 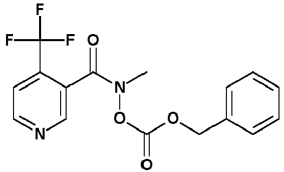 | 1.9 | 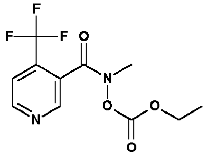 |
| 1.10 | 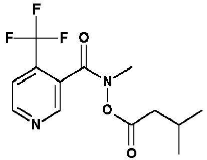 | 1.11 | 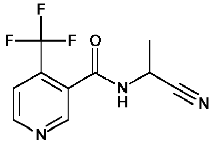 | 1.12 | 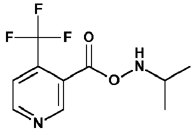 |
| 1.13 | 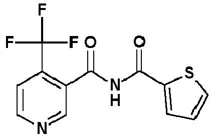 | 1.14 | 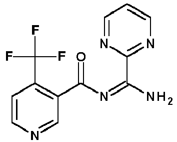 | 1.15 | 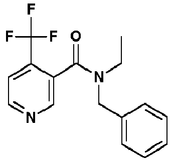 |
| 1.16 | 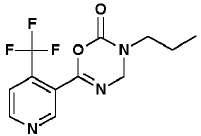 | 1.17 | 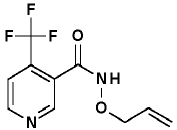 | 1.18 | 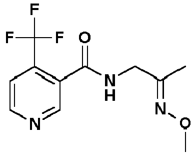 |
| 1.19 | 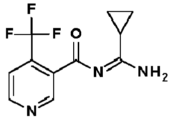 | 1.20 | 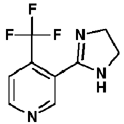 | 1.21 | 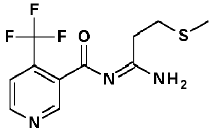 |
| 1.22 | 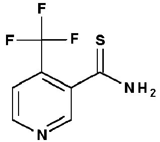 | 1.23 | 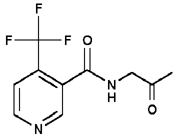 | 1.24 | 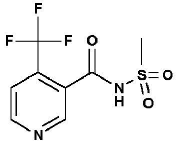 |
| 1.25 | 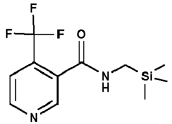 | 1.26 | 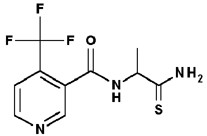 | 1.27 | 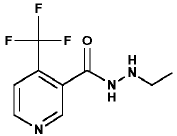 |
| 1.28 | 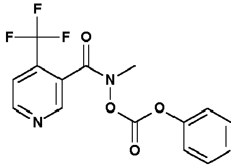 | 1.29 | 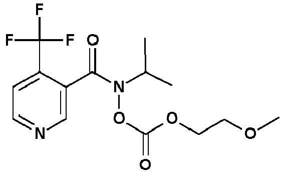 | | |

Accordingly, in a first aspect the present invention provides for the use of one or more 4-(trifluoromethyl)pyridine compounds selected from Table 1 for controlling nuisance, disease carrying or haematophagous dipteran, triatominae or cimicidae insects pests, in particular such haematophagous insect pests (including mosquitoes) by knockdown or by blood feed inhibition.

Nuisance, disease carrying or haematophagous dipteran, triatominae and/or cimicidae insects pests are sometimes referred to herein as "target insects" in singular or plural depending on the context.

In a second aspect, the present invention provides compositions, products, and treated articles (such as non-living material substrates and other non-living materials or non-living target insect loci) comprising a 4-(trifluoromethyl)pyridine compound selected from the group consisting of the compounds shown in Table 1. In particular, a knockdown or blood feed inhibiting effective amount of a 4-(trifluoromethyl)pyridine compound selected from the group consisting of the compounds shown in Table 1 is utilized.

In a third aspect, the present invention provides integrated target insect (incl. mosquito) management or control solutions comprising one or more 4-(trifluoromethyl)pyridine compounds as shown in Table 1.

In a fourth aspect, a method of controlling target insect pests, preferably mosquito vectors of pathogenic disease, which comprises contacting a target insect pest or its environment with a composition comprising a knockdown or blood feed inhibiting effective amount of a 4-(trifluoromethyl)pyridine compound selected from the group consisting of (1.1) - (1.29) is made available.

In one embodiment, suitable targets for such first through fourth aspects include dipteran, triatominae or cimicidae pests include flies, mosquitoes, kissing bugs and bed bugs, especially such pests which are vectors of pathogenic or allergic disease.

In another embodiment, one or more of the 4-(trifluoromethyl)pyridine compounds 1.3, 1.5, 1.6, 1.14, 1.17, 1.19 and 1.21 are utilized in such first through fourth aspects.

Unless otherwise specified, general reference to 4-(trifluoromethyl)pyridine compounds herein pertains to at least one 4-(trifluoromethyl)pyridine compound of Table 1 useful in accordance with such first through fourth and aspects as further detailed herein.

In addition, unless otherwise specified, general reference to active compound compositions herein pertains to compositions comprising at least one 4-(trifluoromethyl)pyridine compound of Table 1 useful in accordance with such first through fourth and aspects as further detailed herein.

In another embodiment, such active compound compositions comprise one or more of the 4-(trifluoromethyl)pyridine compounds 1.3, 1.5, 1.6, 1.14, 1.17, 1.19 and 1.21 that are utilized in such first through fourth aspects

In yet another aspect, the 4-(trifluoromethyl)pyridine compounds selected from the group consisting of compounds 1.5, 1.7, 1.11, 1.12, 1.15, 1.18, 1.20, 1.26, and 1.27 are provided.

In another embodiment, the foregoing aspects are suitable for causing knockdown or blood feed inhibition of a dipteran, triatominae or cimicidae insect pest when carried out in accordance with the present invention.

As well as the biological efficacy of the 4-(trifluoromethyl)pyridine compounds of the present invention against a dipteran, triatominae or cimicidae insect pest (incl. moqusitos and resistant strains of such mosquitos), other considerations for selecting a suitable 4-(trifluoromethyl)pyridine compound could include its safety (such as its toxicity, persistence) to the environment, including to the users of a vector control solution; its suitability for making a vector control solution product (whether indoor residual spray formulation, mosquito net, or another type), its suitability for adherence and availability on a surface over a period of time (in the event the solution is an indoor residual spray), and also its suitability for incorporation into a polymer product (such as a net) so that the compound would be readily available to control mosquitos on the surface of the net over a period of time and the nets can withstand multiple washings.

In an embodiment of each aspect of the present invention involving a vector control solution, the development of vector-borne diseases may be reduced by the control of the dipteran, triatominae or cimicidae insect pest, in particular by mosquito control by knockdown or by blood feed inhibition.

The 4-(trifluoromethyl)pyridine compounds useful in the methods and other aspects of the invention can be prepared similar to known procedures.

In general, the 4-(trifluoromethyl)pyridine compounds useful in the methods, embodiments and other aspects of the invention can be prepared similar to known procedures such as those published in US patent no. 5,360,806.

For example, the 4-(trifluoromethyl)pyridine compounds of Table 1 including compounds 1.2, 1.3, 1.4, 1.6, 1.11, 1.15, 1.18, 1.22, 1.23, 1.24, 1.25, 1.26, and 1.27 can be prepared analogously to procedures published in US patent no. 5,360,806.

For example, the 4-(trifluoromethyl)pyridine compound 1.1, can be prepared analogously to procedures published in WO 9857969.

For example, the 4-(trifluoromethyl)pyridine compounds 1.8, 1.9, 1.10, 1.28, and 1.29 can be prepared analogously to procedures published in WO 2014023531.

For example, the 4-(trifluoromethyl)pyridine compound 1.13 can be prepared analogously to procedures published in WO 2013127768.

For example, the 4-(trifluoromethyl)pyridine compound 1.14, 1.19, and 1.21 can be prepared analogously to procedures published in WO 2013127780.

For example, the 4-(trifluoromethyl)pyridine compound 1.16 can be prepared analogously to procedures published in WO 2001014373.

For example, the 4-(trifluoromethyl)pyridine compound 1.17 can be prepared analogously to procedures published in WO 2001009104.

For example, the 4-(trifluoromethyl)pyridine compound 1.20 can be prepared as described for the analogous unsubstituted pyridine derivative in P. Gogoi, and D. Konwar, Tetrahedron Lett., 2006, 47(1), 79-82.

For example, the 4-(trifluoromethyl)pyridine compounds 1.5, 1.7 and 1.12 can be prepared as described in the procedures shown in the Preparation Examples provided below.

The the 4-(trifluoromethyl)pyridine compounds, active compound compositions and methods of the invention are particularly suitable for the control of mosquitoes including mosquito vectors of human or mammalian pathogenic disease. Mosquito vector control is any method to limit or eradicate mosquito species which transmit disease pathogens. The most frequent types of mosquito vector control employ a variety of strategies.

Mosquito vector control focuses on utilizing preventative methods to control or eliminate mosquito populations. Common preventative measures are
- habitat control - removing or reducing areas where mosquitoes can easily breed can help limit population growth. For example, stagnant water removal, destruction of old tires and cans which serve as mosquito breeding environments and good management of stored water can reduce areas of excessive mosquito incidence.
- reducing contact - limiting exposure to mosquitoes can reduce infection risks significantly. For example, bed nets, window screens on homes, or protective clothing can help reduce the likelihood contact with mosquitoes. To be effective this requires education and promotion of methods among the population to raise the awareness of mosquito threats.
- chemical control - insecticides, larvicides, and repellents can be used to control mosquitoes. For example, larvicides can be used in mosquito breeding zones; insecticides can be applied to house walls or bed nets, and use of personal repellents can reduce incidence of mosquitoes bites and thus infection. The use of pesticides for mosquito vector control is promoted by the World Health Organization (WHO) and has proven to be highly effective.
- biological control - the use of natural mosquito vector predators, such as bacterial toxins or botanical compounds, can help control mosquito populations. Using fish that eat mosquito larvae, has been demonstraited to have some success.
- population control through the release of sterilized, or genetically modified, male mosquitoes has also been shown to control mosquito vector populations and reduce infection risks.

A number of considerations is taken into account when determining which 4-(trifluoromethyl)pyridine compound would be suitable for use in a particular mosquito vector control strategy, such as favourable safety profile, biological performance and affordability.

In one embodiment, a compound selected from the 4-(trifluoromethyl)pyridine compounds shown in Table 1 in accordance with the methods and other aspects of the present invention are useful in controlling mosquitoes, in particular mosquitoes selected from the genus *Anopheles, Culex* and *Aedes.* Examples include *Aedes aegypti, Aedes albopictus, Aedes japonicas, Aedes vexans, Coquillettidia perturbans, Culex molestus, Culex pallens, Culex pipiens, Culex quinquefasciatus, Culex restuans, Culex tarsalis, Anopheles albimanus, Anopheles albitarsis, Anopheles annularis, Anopheles aquasalis, Anopheles arabiensis, Anopheles aconitus, Anopheles atroparvus, Anopheles balabacensis, Anopheles* coluzzii, *Anopheles culicifacies, Anopheles darlingi, Anopheles dirus, Anopheles farauti, Anopheles flavirostris, Anopheles fluviatilis, Anopheles freeborni, Anopheles funestus, Anopheles gambiae s.l., Anopheles koliensis, Anopheles labranchiae, Anopheles lesteri, Anopheles leucosphyrus, Anopheles maculatus, Anopheles marajoara, Anopheles melas, Anopheles merus, Anopheles messeae, Anopheles minimus, Anopheles moucheti, Anopheles nili, Anopheles nuneztovari, Anopheles plumbeus, Anopheles pseudopunctipennis, Anopheles punctipennis, Anopheles punctulatus, Anopheles quadrimaculatus, Anopheles sacharovi, Anopheles sergentii, Anopheles sinensis, Anopheles stephensi, Anopheles subpictus, Anopheles sundaicus, Anopheles superpictus,* and *Mansonia titillans, Ochlerotatus stimulans, Ochlerotatus japonicas* (each of which is an example of a mosquito capable of carrying or vectoring a pathogenic disease).

By control is meant that a 4-(trifluoromethyl)pyridine compound and active compound compositions useful in the methods and other aspects of the invention is employed in a manner that causes knockdown or blood feeding inhibition of the target insect and, in particular, mosquito pest such that biting does not occur or in a manner that decreases pest populations such that biting does not occur as frequently.

In one embodiment, a 4-(trifluoromethyl)pyridine compound as shown in Table 1 useful in the above-noted methods and other aspects of the invention cause symptoms as soon as they enter the target insect and, in particular, mosquito insect pest and are considered extremely fast-acting, causing rapid "knockdown".

In one embodiment, by knockdown is meant a rapid immobilisation or disability of the target insect and, in particular, mosquito insect affected by a 4-(trifluoromethyl)pyridine compound as shown in Table 1 resulting in an induced incapacity for coordinated movement such as flight, walking and/or inability to blood feed such as taking a blood meal.

In another embodiment, by knockdown is meant a state of intoxication and partial paralysis of the target insect and, in particular, mosquito insect affected by a 4-(trifluoromethyl)pyridine compound as shown in Table 1 in a manner which may precede or increase the susceptibility of such insect to being killed.

In a particular embodiment, by control is meant that a 4-(trifluoromethyl)pyridine compound as shown in Table 1 causes rapid "knockdown" or blood feed inhibition of the mosquito pest when used in accordance with the invention.

When the target insect is a mosquito, such control means that biting does not occur or means that mosquito populations are decreased such that biting does not occur as frequently.
In one embodiment, a 4-(trifluoromethyl)pyridine compound as shown in Table 1 useful in the methods and other aspects of the invention cause symptoms as soon as they enter the mosquito and are considered extremely fast-acting, causing rapid "knockdown" or blood feed inhibition.

In an embodiment, pyridine compounds selected from the 4-(trifluoromethyl)pyridine compounds as shown in Table 1 are useful in controlling one or more mosquitos selected from the genus *Anopheles, Culex* and *Aedes,* in particular one or more of *Aedes aegypti, Aedes albopictus, Aedes japonicas, Aedes vexans, Culex molestus, Culex pallens, Culex pipiens, Culex quinquefasciatus, Culex restuans, Culex tarsalis, Anopheles albimanus, Anopheles arabiensis, Anopheles darlingi, Anopheles dirus, Anopheles funestus, Anopheles gambiae s.l., Anopheles melas, Anopheles minimus, Anopheles sinensis, Anopheles stephensi, Mansonia titillans.*

In an embodiment, the 4-(trifluoromethyl)pyridine compounds of Table 1 are useful in the methods and other aspects of the invention to control adult mosquitoes.

Insecticide resistant mosquito species have also been detected and accordingly in an embodiment, a 4-(trifluoromethyl)pyridine compound of Table 1 useful in the methods and other aspects of the invention is suitable for controlling insecticide-resistant mosquitoes, such as pyrethroid, carbamate and/or organophosphate-resistant mosquitoes.

Such mosquito insecticide knockdown resistance is widespread and typically can be either metabolic (i.e., confers resistance to certain pyrethroids) or target-site-based (i.e., extends to all pyrethroids). Quite notably, such knockdown resistance can be mitigated by the methods and other aspects of the invention when otherwise insecticide resistant mosquitoes that are exposed to a 4-(trifluoromethyl)pyridine compound of Table 1 may be more susceptible to being controlled.

Pyrethroids are the only insectides that have obtained WHO recommendation against Malaria vectors on both Indoor Residuals Sprays (IRS) and Long Lasting Insecticidal Mosquito Nets (LLINs), in the form of alpha-cypermethrin, bifenthrin, cyfluthrin, permethrin, deltamethrin, lambda-cyhalothrin and etofenprox. It has been the chemical class of choice in agriculture and public health applications over the last several decades because of its relatively low toxicity to humans, rapid knock-down effect, relative longevity (duration of 3-6 months when used as IRS), and low cost. However, massive use of pyrethroids in agricultural applications and for vector control led to the development of resistance in major malaria and dengue vectors. Strong resistance has e.g. been reported for the pyrethroid Deltamethrin (and Permethrin) for the *Anopheles gambiae* Tiassalé (from southern Cote d'lvoire) strain (Constant V.A. Edi et al., Emerging Infectious Diseases; Vol. 18, No. 9, September 2012). Pyrethroid resistance was also reported for Permethrin, Deltamethrin and Lambda-Cyhalothrin for the *Aedes aegypti* Cayman Island strain (Angela F. Harris et al., Am. J. Trop. Med. Hyg., 83(2), 2010) and Alpha- Cypermethrin, Permethrin and Lambda-Cyhalothrin for certain *Anopheles* strains (Win Van Bortel, Malaria Journal, 2008, 7:102).

In another embodiment of the invention, the 4-(trifluoromethyl)pyridine compounds of Table 1 can be suitable for use against insecticide-resistant mosquitoes that are selected from *Anopheles gambiae* RSPH, *Anopheles gambiae* Tiassalé, *Anopheles gambiae* Akron, *Anopheles gambiae* Kisumi Rdl, *Anopheles arabiensis* NDjamina, *Anopheles gambiae* VK7, *Anopheles funestus* FUMOZ-R, *Aedes aegypti* Grand Cayman and *Culex quinquefasciatus* strain POO.

*Anopheles gambiae,* strain RSPH is a multi-resistant mosquito (target-site and metabolic-resistance) that is described in the reagent catalog of the Malaria Research and Reference Reagent Resource Center (www.MR4.org; MR4-number: MRA-334).

*Anopheles gambiae,* strain Tiassalé is a multi-resistant mosquito (target and metabolic-resistant strain) which shows cross-resistance between carbamates, organophosphates and pyrethroids and is described in Constant V.A. Edi et al., Emerging Infectious Diseases; Vol. 18, No. 9, September 2012 and Ludovic P Ahoua Alou et al., Malaria Journal 9: 167, 2010).

*Anopheles gambiae,* strain AKRON is a multi-resistant mosquito (target and metabolic-resistant strain) and is described in Djouaka F Rousseau et al., BMC Genomics, 9:538; 2008.

*Anopheles coluzzii,* strain VK7 is a target-resistant mosquito and is described in Dabire Roch Kounbobr et al., Malaria Journal, 7: 188, 2008.

*Anopheles funestus,* strain FUMOZ is a metabolic -resistant strain and is described in Hunt et al., Med Vet Entomol. 2005 Sep; 19(3):271-5). In this article it has been reported that *Anopheles funestus* - as one of the major malaria vector mosquitoes in Africa - showed resistance to pyrethroids and carbamate insecticides in South Africa.

*Anopheles gambiae,* strain Kisumu Rdl, a dieldrin resistant strain from Kenya.

*Anopheles arabiensis,* strain NDjamina, a pyrethroid resistant from Chad.

*Aedes aegypti,* strain Grand Cayman is a target-resistant mosquito and is described in Angela F. Harris, Am. J. Tro. Med. Hyg. 83(2), 2010.

*Culex quinquefasciatus* (metabolic -resistant to DDT strain P00); received from Texchem, Penang, Malaysia.

Vector control solution are means to control a target insect vector, such as a mosquito. Examples of such means are compositions, products, and treated articles, which include a non-living substrate or non-living material incorporating (e.g. coated or impregnated with) at least one 4-(trifluoromethyl)pyridine compound of Table 1, as well as spray products (e.g. indoor sprays, and aerosol products) comprising a 4-(trifluoromethyl)pyridine compound of Table 1, paint compositions comprising a 4-(trifluoromethyl)pyridine compound of Table 1, and products or treated articles comprising at least one 4-(trifluoromethyl)pyridine compound of Table 1.

Examples of integrated target insect, esp. mosquito vector management or control solutions of the invention, such as solutions for controlling mosquito bites or decreasing relevant mosquito populations, include the use of such compositions, products, treated articles and non-living substrates of the invention at a locus of potential or known interaction between the mosquito vector and an animal, including a human, that is susceptible to a pathogenic disease infection transmitted by such vector. Suitable integrated solutions within the scope of the present invention also include identifying mosquito breeding sites and positioning such compositions, products, treated articles and non-living substrates of the invention at such sites.

Examples of a non-living substrate or non-living material of the invention are self-supporting film/sheet (e.g., screens), threads, fibres, yarns, pellets, weaves (or textiles (e.g. for clothing)), nets, tents, and curtains incorporating (e.g. coated or impregnated with) at least one 4-(trifluoromethyl)pyridine compound of Table 1, which can be used to protect against mosquito bites. In particular, it is well known that humans can be protected in their sleep from mosquito bites by insecticidally coated sleeping nets. Coated or impregnated weaves of the invention can also be used as curtains in front of windows, doors open eaves, or ventilation openings, in order to control mosquito entering dwellings.

The use of at least one 4-(trifluoromethyl)pyridine compound of Table 1 in a non-living material or substrate of the present invention (e.g. nets and weaves) achieves at least one of the following objects:
- good insecticidal effect
- fast-acting insecticidal efficacy
- long-lasting insecticidal efficacy
- uniform release of active ingredient
- long durability (including resisting multiple washings over an extended period)
- simple production
- safe to the user

The nets and weaves (or textiles) of the invention that incorporate (e.g. are coated or impregnated with) at least one 4-(trifluoromethyl)pyridine compound of Table 1, are made up of a variety of natural and synthetic fibres, also as textile blends in woven or non-woven form, as knit goods or fibres. Natural fibres are for example cotton, raffia, jute, flax, sisal, hessian, wool, silk or hemp. Synthetic fibres may be made of polyamides, polyesters, polyacrylonitriles, polyolefines, for example polypropylene or polyethylene, Teflon, and mixtures of fibres, for example mixtures of synthetic and natural fibres. Polyamides, polyolefins and polyesters are preferred as fibre material. Polyester, such a polyethylene terephthalate, polyethylene and polypropylene are especially preferred. Most preferred are nettings made from polyester, polyethylene and/or polypropylene.

The art discloses methods suitable for incorporating (by way of coating) a compound onto nets and weaves (see for example, WO2003/034823, WO 2008/122287, WO 01/37662, US2009036547, WO 2007/036710), from dipping or submerging them into a formulation of the insecticide or by spraying the formulation onto their surfaces. After treating the nets and weaves of the invention, they may be dried simply at ambient temperatures (see also below for more background). Such methods are also suitable for incorporating (by way of coating) at least one 4-(trifluoromethyl)pyridine compound of Table 1.

Also disclosed in the art are methods suitable for incorporating (by way of impregnating) a compound within the net or weave by making polymer material in the presence of the 4-(trifluoromethyl)pyridine and, optionally, other active compounds, which is then extruded into fibres, threads or yarns, for making the nets and weaves (see for example, WO08004711, WO2009/121580, WO2011/128380, WO2011/141260, WO2010/118743). Such nets and weaves having available at the surface of the net and weave an effective amount of at least one 4-(trifluoromethyl)pyridine compound of Table 1 so as to control mosquito bites. Generally the 4-(trifluoromethyl)pyridine of Table 1 compound is mixed with the molten polymer. Such methods are also suitable for incorporating (by way of impregnating) at least one 4-(trifluoromethyl)pyridine compound of Table 1.

The term "incorporating" or "incorporated" in context of the compound of the invention, additives and other insecticides is meant that the substrate or non-living material comprises or contains the respectively defined 4-(trifluoromethyl)pyridine compound, additive and/or insecticide, such as by coating or impregnation.

Preferably the substrate of the present invention is a net, which net is preferably a long lasting net, incorporated with at least one 4-(trifluoromethyl)pyridine compound of Table 1 by way of coating the net with a composition comprising such pyridine compounds, or by way of making a polymeric material in the presence of such pyridine compounds and then processing the resultant polymeric material into an inventive net.

In accordance with the invention, when at least one 4-(trifluoromethyl)pyridine compound of Table 1 is used within the polymer, then during use of the resulting net or weave made from the polymer, such pyridine compound is released to the surface of the net to control against mosquito bites - such control is sustained at adequate level and for adequate amount of time.

Examples of suitable polymers are polyamides, polyesters, polyacrylonitriles, polyolefines, such as polyethylene compositions that can be made from different polyethylene polymers; these may be LDPE, LLDPE, MDPE and HDPE. LLDPE (Linear low-density polyethylene) is a substantially linear polymer (polyethylene), with significant numbers of short branches, commonly made by copolymerization of ethylene with longer-chain olefins. MDPE is medium-density polyethylene is a substantially linear polymer of polyethylene with shorter chain length than HDPE. HDPE (High-Density PolyEthylene) or PolyEthylene High-Density (PEHD) is a polyethylene thermoplast. HDPE has little branching, giving it stronger intermolecular forces and tensile strength than lower- density polyethylene. It is also harder and more opaque and can withstand somewhat higher temperatures (120°C /248°F for short periods, 110°C /230°F continuously). HDPE yarns are stronger than LDPE mixed polyethylene yarns. LLDPE differs structurally from conventional low- density polyethylene (LDPE) because of the absence of long chain branching. These polyethylene compositions (HDPE, LDPE, LLDPE and mixture thereof) are generally used for preparing yarns and polyethylene based textile products. Methods for incorporating an insecticide compound into the polymer without weakening its resulting properties are known in the art, such as using mixtures of HDPE and LDPE. Such methods can also be used to incorporate a 4-(trifluoromethyl)pyridine compound of Table 1 into a polymer.

In one embodiment, at least one 4-(trifluoromethyl)pyridine compound of Table 1 is incorporated into a polymer masterbatch by using the foregoing methods to encapsulate such compound during a heat process into a carrier resin such as one of the suitable polmers mentioned above. The masterbatch mixture is is then cooled and typically cut into a granular shape. The masterbatch composition thus prepared is useful for incorporation in to a polymer matrix and facilitates the impartation of insect-resistant properties to raw polymers during the plastics manufacturing process. These insect-resistant materials may then be further extruded to prepare various fabrics or materials which can be formed into nets or weaves having long lasting insecticidal resistance.

Examples of spray products of the present invention are indoor residual sprays or space sprays comprising a 4-(trifluoromethyl)pyridine compound of Table 1. Indoor Residual Spraying (IRS) is the technique of applying a residual deposit of an insecticide onto indoor surfaces where vectors rest, such as on walls and ceilings. The primary goal of indoor residual spraying is to reduce the lifespan of the mosquito vectors and thereby reduce or interrupt disease transmission. The secondary impact is to reduce the density of mosquitoes within the treatment area. IRS is a recognised, proven and cost-effective intervention method for the control of malaria and it is also used in the management of Leishmaniasis and Chagas disease. Many malaria mosquito vectors are endophilic, resting inside houses after taking a blood meal. These mosquitoes are particularly susceptible to control through indoor residual spraying (IRS) comprising a 4-(trifluoromethyl)pyridine compound of Table 1. As its name implies, IRS involves coating the walls and other surfaces of a house with a residual insecticide. In one embodiment, the 4-(trifluoromethyl)pyridine compound will knockdown mosquitoes that come in contact with these surfaces. IRS does not directly prevent people from being bitten by mosquitoes. Rather, it usually controls mosquitoes after they have blood fed, if they come to rest on the sprayed surface. IRS thus prevents transmission of infection to other persons. To be effective, IRS must be applied to a very high proportion of households in an area (usually greater than 70 percent). Although the community plays a passive role in IRS programs, cooperation with an IRS effort is a key to its success. Community participation for IRS often consists of cooperating with the spray teams by removing food and covering surfaces prior to spraying and refraining from covering the treated surfaces with new paint or plaster. However, community or individual householder opposition to IRS due to the smell, mess, possible chemical exposure, or sheer bother has become a serious problem in some areas. Therefore, sprays in accordance with the invention having good residual efficacy and acceptable odour are particularly suited as a component of integrated mosquito vector management or control solutions.

In contrast to IRS, which requires that the active 4-(trifluoromethyl)pyridine compound of Table 1 is bound to surfaces of dwellings, such as walls, ceiling as with a paint, for example, space spray products of the invention rely on the production of a large number of small insecticidal droplets intended to be distributed through a volume of air over a given period of time. When these droplets impact on a target mosquito, they deliver a knockdown effective dose of the 4-(trifluoromethyl)pyridine compound effective to control the mosquito. The traditional methods for generating a space-spray include thermal fogging (whereby a dense cloud of 4-(trifluoromethyl)pyridine droplets is produced giving the appearance of a thick fog) and Ultra Low Volume (ULV), whereby droplets are produced by a cold, mechanical aerosol-generating machine. Ready-to-use aerosols such as aerosol cans may also be mentioned.

Since large areas can be treated at any one time this method is a very effective way to rapidly reduce the population of flying mosquitoes in a specific area. Since there is very limited residual activity from the application it must be repeated at intervals of 5-7 days in order to be fully effective. This method can be particularly effective in epidemic situations where rapid reduction in mosquito numbers is required. As such, it can be used in urban dengue control campaigns.

Effective space-spraying is generally dependent upon the following specific principles:
- Target insects are usually flying through the spray cloud (or are sometimes impacted whilst resting on exposed surfaces). The efficiency of contact between the spray droplets and target insects is therefore crucial. This is achieved by ensuring that spray droplets remain airborne for the optimum period of time and that they contain the right dose of insecticide. These two issues are largely addressed through optimizing the droplet size.
- If droplets are too big they drop to the ground too quickly and don't penetrate vegetation or other obstacles encountered during application (limiting the effective area of application). If one of these big droplets impacts an individual insect then it is also 'overkill' since a high dose will be delivered per individual insect.
- If droplets are too small then they may either not deposit on a target insect (no impaction) due to aerodynamics or they can be carried upwards into the atmosphere by convection currents.
- The optimum size of droplets for space-spray application are droplets with a Volume Median Diameter (VMD) of 10-25 microns.

The active compound compositions of the present invention comprising at least one 4-(trifluoromethyl)pyridine compound of Table 1 may be made available in a spray product as an aerosol-based application, including aerosolized foam applications. Pressurised cans are the typical vehicle for the formation of aerosols. An aerosol propellant that is compatible with the particular 4-(trifluoromethyl)pyridine compound is used. Preferably, a liquefied-gas type propellant is used. Suitable propellants include compressed air, carbon dioxide, butane and nitrogen. The concentration of the propellant in the active compound composition is from about 5 percent to about 40 percent by weight of the pyridine composition, preferably from about 15 percent to about 30 percent by weight of such 4-(trifluoromethyl)pyridine containing composition.

In one embodiment, the such 4-(trifluoromethyl)pyridine containing formulations of the invention can also include one or more foaming agents. Foaming agents that can be used include sodium laureth sulphate, cocamide DEA, and cocamidopropyl betaine. Preferably, the sodium laureth sulphate, cocamide DEA and cocamidopropyl are used in combination. The concentration of the foaming agent(s) in the acitive compound composition is from about 10 percent to about 25 percent by weight, more preferably 15 percent to 20 percent by weight of the composition.

When such formulations are used in an aerosol application not containing foaming agents, the active compound compositions of the present invention can be used without the need for mixing directly prior to use. However, aerosol formulations containing the foaming agents do require mixing (i.e. shaking) immediately prior to use. In addition, if the formulations containing foaming agents are used for an extended time, they may require additional mixing at periodic intervals during use.

A dwelling area may also be treated with an active compound composition of the present invention by using a burning formulation, such as a candle, a smoke coil or a piece of incense containing the composition. For example, composition may be comprised in household products such as "heated" air fresheners in which insecticidal compositions are released upon heating, for example, electrically, or by burning.

The active compound compositions of the present invention containing a 4-(trifluoromethyl)pyridine compound of Table 1 may be made available in a spray product as an aerosol, a mosquito coil, and/or a vaporiser or fogger.

The concentration of the a 4-(trifluoromethyl)pyridine compound of Table 1 in the polymeric material, fibre, yarn, weave, net, or substrate, each of the invention, can be varied within a relatively wide concentration range from, for example 0.05 to 15 percent by weight, preferably 0.2 to 10 percent by weight, more preferably 0.4 to 8 percent by weight, especially 0.5 to 5, such as 1 to 3, percent by weight.

The percentages mentioned above are based on dry weight of the net or substrate or non-living material.

Similarly, the concentration of the 4-(trifluoromethyl)pyridine compound of Table 1 in the composition of the invention (whether for treating surfaces or for coating a fibre, yarn, net, weave) can be varied within a relatively wide concentration range from, for example 0.1 to 70 percent by weight, such as 0.5 to 50 percent by weight, preferably 1 to 40 percent by weight, more preferably 5 to 30 percent by weight, especially 10 to 20 percent by weight.

The concentration shall be chosen according to the field of application such that the requirements concerning knockdown efficacy, durability and toxicity are met. Adapting the properties of the material can also be accomplished and so custom-tailored textile fabrics are obtainable in this way.

The 4-(trifluoromethyl)pyridine compounds of Table 1 (Al) when used in the IRS methods of the invention is present on a surface of a dwelling at a coverage of from 0.01 to 2 grams of Al per m2, preferably from 0.05 to 1 grams of Al per m2, especially from 0.1 to 0.7 grams of Al per m2.

Accordingly an effective amount of a 4-(trifluoromethyl)pyridine compound of Table 1 can depend on the specific use pattern, the mosquito against which control is most desired and the environment in which 4-(trifluoromethyl)pyridine compound of Table 1 will be used. Therefore, an effective amount of a 4-(trifluoromethyl)pyridine compound of Table 1 is sufficient that control of a mosquito is achieved; in case of:
- use as IRS formulation, the effective amount is such that coverage of the Al on the surface is from 0.01 to 2 grams of Al per m2, preferably from 0.05 to 1 grams of Al per m2, especially from 0.1 to 0.7 grams of Al per m2;
- use incorporatated within a net or substrate, the effective amount is 0.05 to 15 percent by weight, preferably 0.2 to 10 percent by weight, more preferably 0.4 to 8 percent by weight, especially 0.5 to 5, such as 1 to 3, percent by weight.

Generally the 4-(trifluoromethyl)pyridine compound of Table 1 when used in certain products of the invention is continuously distributed in a thread, yarn, net or weave, but can also be partially or discontinuously distributed in a thread, yarn, net or weave. For example, a net may contain certain parts which are coated or which is made-up of impregnated fibre, and certain other parts which are not; alternatively some of the fibres making up the net is impregnated, or is coated, with the compound of the invention, and some of the other fibres not or these other fibres are impregnated, or are coated, with another active compound such as an insecticide compound (see below).

Nets of the invention impregnated, or coated, with a 4-(trifluoromethyl)pyridine compound of Table 1 can satisfy the criteria of the WHOPES directive (see "Guidelines for laboratory and field testing of long-lasting insecticidal mosquito nets", 2005, http://www.who.int/whopes/guidelines/en/) for insecticide-containing long-lasting mosquito nets up to 20 washes only, which means that such nets should not lose their biological activity after just 20 wash cycles or so.

In an embodiment, a net of the invention impregnated, or coated, with 4-(trifluoromethyl)pyridine compound of Table 1 can have biological activity in accordance with WHOPES guidelines of a knockdown after 60 minutes of between 95 percent and 100 percent or a mortality after 24 hours of between 80 percent and 100 percent after at least 20, such as 25, preferably at least 30 and even more preferably at least 35 washes.

The "WHOPES directive" is to be understood as meaning the directive "Guidelines for laboratory and field testing of long-lasting insecticidal mosquito nets", 2005). This directive is retrievable at the following interact address: http://www.who.int/whopes/guidelines/en/.

When a net is "impregnated with" a 4-(trifluoromethyl)pyridine compound of Table 1 to prepare a net of the present invention, the fibres making up the net are made by melting a polymer, a 4-(trifluoromethyl)pyridine compound of Table 1 and optionally other compounds, such as other insecticides, additives, stabilisers. When a net is impregnated with such a 4-(trifluoromethyl)pyridine compound, then the net of the invention contains synthetic fibres; in contrast, a net of the invention coated with such a 4-(trifluoromethyl)pyridine compound contains synthetic fibres and/or natural fibres.

The polymeric materials useful in the compositions of the invention incorporating at least one 4-(trifluoromethyl)pyridine compound of Table 1 can be produced by mixing such a pyridine compound with the polymer in the liquid phase, and optionally other additives (such as binders and/or synergists), and other insecticidal compounds.

Methods of making suitable polymeric materials and then processing it are described in the art - see for example, WO09121580, WO2011/141260.

For example, nets based on a 4-(trifluoromethyl)pyridine insecticide-containing polymeric material are produced by the following steps:
a) melting the polymer to be used and one or more insecticidally active ingredients together or separately at temperatures between 120 and 250°C,
b) forming the melt of step a) into spun threads and cooling,
c) optionally leading the spun threads formed in step b) through a drawing system and drawing and then optionally setting out the threads,
d) knitting the spun threads to form a net,
e) subjecting the net to a heat-setting operation wherein the temperature for the heat-setting operation is chosen to be 20°Cbelow the melting temperature of the polymer to be used.

The heat setting in step e) of the production of the nets is preceded by a washing step. Water and a detergent is preferably used for this. The heat setting is preferably carried out in a dry atmosphere.

Although the manufacture of the nets incorporated with the insecticide compound can occur in a single location, it is also envisaged that the different steps can take place in different locations. So a composition comprising a 4-(trifluoromethyl)pyridine compound may be made which can then be processed into a polymer. Accordiingly, the present invention also provides a composition comprising a 4-(trifluoromethyl)pyridine compound of Table 1 in a concentrated form, which composition may also contain additives (such as binders and/or synergists), and other insecticidal compound(s) (which composition had been prepared explicitly for making a polymer material impregnated with the 4-(trifluoromethyl)pyridine compound of Table 1 (such a composition is often referred to as a "masterbatch")). The amount of the 4-(trifluoromethyl)pyridine compound of Table 1 in the masterbatch would depend on the circumstances, but in general can be 10 to 95 percent by weight, such as 20 to 90 percent by weight, preferably 30 to 85 percent by weight, more preferably 35 to 80 percent by weight, especially 40 to 75 percent by weight.

Also made available in the present invention are compositions or formulations for coating walls, floors and ceilings inside of buildings and for coating a substrate or non-living material, which comprise a 4-(trifluoromethyl)pyridine compound of Table 1. The inventive compositions can be prepared using known techniques for the purpose in mind, which could contain a binder to facilitate the binding of the compound to the surface or other substrate. Agents useful for binding are known in the art and tend to be polymeric in form. The type of binder suitable for composition to be applied to a wall surface having particular porosities, binding characteristics would be different to a fibre, yarn, weave or net - a skilled person, based on known teachings, would select a suitable binder.

Typical binders are poly vinyl alcohol, modified starch, poly vinyl acrylate, polyacrylic, polyvinyl acetate co polymer, polyurethane, and modified vegetable oils. Suitable binders can include latex dispersions derived from a wide variety of polymers and co-polymers and combinations thereof. Suitable latexes for use as binders in the inventive compositions comprise polymers and copolymers of styrene, alkyl styrenes, isoprene, butadiene, acrylonitrile lower alkyl acrylates, vinyl chloride, vinylidene chloride, vinyl esters of lower carboxylic acids and alpha, beta-ethylenically unsaturated carboxylic acids, including polymers containing three or more different monomer species copolymerized therein, as well as post-dispersed suspensions of silicones or polyurethanes. Also suitable may be a polytetrafluoroethylene (PTFE) polymer for binding the active ingredient to other surfaces.

The formulation according to the present invention comprises at least one 4-(trifluoromethyl)pyridine compound listed in Table 1 (or a pesticide (A)), and a carrier, such as water (C), and optionally a polymeric binder (B) and further components (D).

The polymeric binder binds the pyridine compounds to the surface of the non-living material and ensures a long-term effect. Using the binder reduces the elimination of the pyridine pesticide out of the non-living material due to environmental effects such as rain or due to human impact on the non-living material such as washing and/or cleaning it. The further components can be an additional insecticide compound, a synergist, a UV stabiliser.

The inventive compositions can be in a number of different forms or formulation types, such as suspensions, capsules suspensions, and a person skilled in the art can prepare the relevant composition based on the properties of the particular 4-(trifluoromethyl)pyridine compound, its uses and also application type.

For example, the 4-(trifluoromethyl)pyridine compounds used in the methods, embodiments and other aspects of the present invention may be encapsulated in the formulation. A encapsulated compound can provide improved wash-fastness and also longer period of activity. The formulation can be organic based or aqueous based, preferably aqueous based.

Microencapsulated 4-(trifluoromethyl)pyridine compounds suitable for use in the compositions and methods according to the invention are prepared with any suitable technique known in the art. For example, various processes for microencapsulating material have been previously developed. These processes can be divided into three categories-physical methods, phase separation and interfacial reaction. In the physical methods category, microcapsule wall material and core particles are physically brought together and the wall material flows around the core particle to form the microcapsule. In the phase separation category, microcapsules are formed by emulsifying or dispersing the core material in an immiscible continuous phase in which the wall material is dissolved and caused to physically separate from the continuous phase, such as by coacervation, and deposit around the core particles. In the interfacial reaction category, microcapsules are formed by emulsifying or dispersing the core material in an immiscible continuous phase and then an interfacial polymerization reaction is caused to take place at the surface of the core particles. The concentration of the pyridine compound present in the microcapsules can vary from 0.1 to 60% by weight of the microcapsule.

The formulation used in the 4-(trifluoromethyl)pyridine compositions, methods, embodiments and other aspects according to the invention may be formed by mixing all ingredients together with water optionally using suitable mixing and/or dispersing aggregates. In general, such a formulation is formed at a temperature of from 10 to 70°C, preferably 15 to 50 °C, more preferably 20 to 40°C In general, it is possible to use a 4-(trifluoromethyl)pyridine compound of Table 1 (as pesticide) (A), solid polymer (B) and optionally additional additives (D) and to disperse them in the aqueous component (C)

If a binder is present in a composition of the present invention, it is preferred to use dispersions of the polymeric binder (B) in water as well as aqueous formulations of the pyridine pesticide (A) in water which have been separately prepared before. Such separate formulations may contain additional additives for stabilizing (A) and/or (B) in the respective formulations and are commercially available. In a second process step, such raw formulations and optionally additional water (component (C)) are added.

Also combinations are possible, i.e. using a pre-formed dispersion of (A) and/or (B) and mixing it with solid (A) and/or (B).

A dispersion of the polymeric binder (B) may be a pre-manufactured dispersion already made by a chemicals manufacturer.

However, it is also within the scope of the present invention to use "hand-made" dispersions, i.e. dispersions made in small-scale by an end-user. Such dispersions may be made by providing a mixture of about 20 percent of the binder (B) in water, heating the mixture to temperature of 90 to 100°C and intensively stirring the mixture for several hours.

It is possible to manufacture the formulation as a final product so that it can be readily used by the end-user for the process according to the present invention.

However, it is of course also possible to manufacture a concentrate, which may be diluted by the end-user with additional water (C) to the desired concentration for use.

In an embodiment, a composition suitable for IRS application or a coating formulation containing a 4-(trifluoromethyl)pyridine compound of Table 1 contains the active ingredient and a carrier, such as water, and may also one or more co-formulants selected from a dispersant, a wetter, an anti-freeze, a thickener, a preservative, an emulsifier and a binder or sticker.

The 4-(trifluoromethyl)pyridine compound of Table 1 is generally milled to a desired particle size, such as the particle size distribution d(0.5) is generally from 3 to 20, preferably 5 to 15, especially 7 to 12, µm.

Furthermore, it may be possible to ship the formulation to the end-user as a kit comprising at least
- a first component comprising at least one 4-(trifluoromethyl)pyridine compound listed in Table 1 (A); and
- a second component comprising at least one polymeric binder (B).
- Further additives (D) may be a third separate component of the kit, or may be already mixed with components (A) and/or (B).

The end-user may prepare the formulation for use by just adding water (C) to the components of the kit and mixing.

The components of the kit may also be formulations in water. Of course it is possible to combine an aqueous formulation of one of the components with a dry formulation of the other component(s).

As an example, the kit can comprise
- one formulation of a 4-(trifluoromethyl)pyridine compound listed in Table 1 (A) and optionally water (C); and
- a second, separate formulation of at least one polymeric binder (B), water as component (C) and optionally components (D).

Accordingly, in a further aspect the present invention provides a kit for treating a fibre, yarn, net and weave by coating wash resistant insecticidal properties thereto comprising: a first sachet comprising a pre-measured amount of at least one 4-(trifluoromethyl)pyridine compound listed in Table 1, and a second sachet comprising a pre-measured amount of at least one polymeric binder. The resulting treated fibre, yarn, net and weave has imparted thereto the insecticidal properties needed for vector control, such as to control vector-carrying mosquitoes.

The concentrations of the components (A), (B), (C) and optionally (D) will be selected by the skilled artisan depending of the technique to be used for coating/ treating.

In general, the amount of pyridine pesticide (A) may be up to 50, preferably 5 to 50, such as 10 to 40, especially 15 to 30, percent by weight, based on weight of the composition.

The amount of polymeric binder (B) may be in the range of 0.01 to 30, preferably 0.5 to 15, more preferably 1 to 10, especially 1 to 5, percent by weight, based on weight of the composition.

If present, in general the amount of additional components (D) is from 0.1 to 20, preferably 0.5 to 15, percent by weight, based on weight of the composition. If present, suitable amounts of pigments and/or dyestuffs are in general 0.01 to 5, preferably 0.1 to 3, more preferably 0.2 to 2, percent by weight, based on weight of the composition.

A typical formulation ready for use comprises 0.1 to 40, preferably 1 to 30, percent of components (A), (B), and optionally (D), the residual amount being water (C).

A typical concentration of a concentrate to be diluted by the end-user may comprise 5 to 70, preferably 10 to 60, percent of components (A), (B), and optionally (D), the residual amount being water (C).

The formulation of the present invention may be applied to polymeric material before their formation into the required products, e.g. while still a yarn or in sheet form, or after formation of the relevant products.

For the case of nets and/or weaves, a process for coating nets and/or weaves at least comprising the following steps:
a) treating the nets and/or weaves with the aqueous formulation according to the invention by any of the procedural steps selected from the group of
   (a1) passing the material through the formulation; or
   (a2) contacting the material with a roller that is partly or fully dipped into the formulation and
   drawing the formulation to the side of the material in contact with the roller, or
   (a3) submerging the material into the formulation; or
   (a4) spraying the formulation onto the material; or
   (a5) brushing the formulation onto or into the material; or
   (a6) applying the formulation as a foam; or
   (a7) coating the formulation onto material.
b) optionally removing surplus formulation by squeezing the material between rollers or by means of a doctor blade; and
c) drying the material.

In case the raw materials containing residues of preceding production processes, e.g. sizes, spin finishes, other auxiliaries and/or impurities, it may be beneficial to perform a washing step before the coating.

Specifically, the following details are important for the steps a), b), and c).

### Step a1)

The formulation is applied by passing the material through the aqueous formulation. Said step is known by a person skilled in the art as padding. In a preferred embodiment the material is completely submerged in the aqueous formulation either in a trough containing the liquor or the material is passed through the formulation which is held between two horizontally oriented rollers. In accordance with the invention, the material may either be passed through the formulation or the formulation may be passed through the material. The amount of uptake of the formulation will be influenced by the stability of concentrated baths, the need for level distribution, the density of material and the wish to save energy costs for drying and curing steps. Usual liquor-uptakes may be 40 to 150 percent on the weight of material. A person skilled in the art is familiar with determining the optimum value. Step a1) is preferred for coating open-width material which is later tailored into nets.

For small-scale production or re-coating of non-treated nets, use of a simple hand-held roller may be sufficient.

### Step a2)

It is further possible to apply the aqueous formulation on the material by a roller that is partly dipped into the dispersion thus applying the dispersion to the side of the material in contact with the roller (kiss-rolling). By this method it is possible to coat only one side of the material which is advantageous if e.g. direct contact of the human skin with insecticide-treated material is to be avoided.

Coating of the material in step a1), a2) or a3) is typically carried out at temperatures from 10 to 70 degrees centigrade, preferably 15 to 50°C, more preferably 20 to 40°C

### Step a4)

The spray may be applied in continuous processes or in batch-wise processes in suitable textile machines equipped with a spraying device, e.g. in open-pocket garment washer/extractors. Such equipment is especially suitable for impregnating ready-made nets.

### Step a6)

A foam comprises less water than the dispersion mentioned above. The drying process may therefore be very short. The treatment may be performed by injecting gas or blends of gas (e.g., air) into it. The addition of surfactants, preferably with film-forming properties, may be required. Suitable surfactants and the required technical equipment are known to persons skilled in the art.

### Step a7)

A coating process may preferably carried out in a doctor-blade process. The process conditions are known to a person skilled in the art.

### Step b)

The surplus emulsion is usually removed by squeezing the material, preferably by passing the material through rollers as known in the art thus achieving a defined liquor uptake. The squeezed-off liquor may be re-used. Alternatively, the surplus aqueous emulsion or aqueous dispersion may be removed by centrifuging or vacuum suction.

### Step c)

Drying may be performed at ambient temperatures. In particular, such a passive drying may be carried out in hot-dry climate. Of course, the drying process may be accelerated applying elevated temperatures. An active drying process would normally be performed during high scale processing. The drying is in general carried out temperatures below 200°C. Preferred temperatures are from 30 to 170°C , more preferably at room temperature. The temperature choice is determined by the thermal stability of the insecticide in the formulation and the thermal stability of the non-living material impregnated.

For the method according to the invention aqueous formulation comprising at least one pigment and/or at least one dyestuff may be used so that the material is not only coated with the mosquitocidal 4-(trifluoromethyl)pyridine compound but in addition also coloured at the same time.

In a further aspect, the present invention provides a method for treating a fibre, yarn, net and weave by coating wash resistant insecticidal properties thereto comprising (i) preparing a treatment composition, which comprises at least one 4-(trifluoromethyl)pyridine compound listed in Table 1, (ii) treating said fibre, yarn, net and weave and (iii) drying the resulting treated a fibre, yarn, net and weave.

The polymeric binder (B) can be dispersed in an aqueous formulation and comprises one or more fluorinated acrylic copolymers useful in the water and oil resistant formulations includes copolymer prepared by the polymerization of a perfluoroalkyl acrylate monomer and a comonomer, especially an acrylate monomer. The binder may also be fluorocarbon resins (as described in WO 2006/128870.

Only water is used as solvent for the formulation. However, trace amounts of organic solvents miscible with water may be present. Examples of solvents comprise water-miscible alcohols, e.g. monoalcohols such as methanol, ethanol or propanol, higher alcohols such as ethylene glycol or polyether polyols and ether alcohols such as butyl glycol or methoxypropanol. Preferably the content of an organic solvent is no more than 5 percent by weight (based on component (C), more preferably no more than 1 percent by weight (based on component (C), in particular no more than 0.1 percent by weight, based on component (C).

Depending on the intended use of the non-living material to be treated with the 4-(trifluoromethyl)pyridine formulation according to the present invention may further comprise one or more components or additives (D) selected from preservatives, detergents, fillers, impact modifiers, anti-fogging agents, blowing agents, clarifiers, nucleating agents, coupling agents, fixative agents, cross-linking agents, conductivity-enhancing agents (antistats), stabilizers such as antioxidants, carbon and oxygen radical scavengers and peroxide decomposing agents and the like, flame retardants, mould release agents, agents having UV protecting properties, spreading agents, antiblocking agents, anti-migrating agents, foam-forming agents, anti-soiling agents, thickeners, further biocides, wetting agents, plasticizers and film-forming agents, adhesive or anti-adhesive agents, optical brightening (fluorescent whitening) agents, pigments and dyestuffs.

A typical amount of the polymeric binder (B) is from 0.01 to 10 percent by weight (dry weight) of the (dry) weight of the material. As a general guideline, the weight ratio between 4-(trifluoromethyl)pyridine compound and binder (B) should approximately be constant with a value depending on the biological activity and migratory ability of the 4-(trifluoromethyl)pyridine compound, i.e. the higher the amount of such compound the higher also the amount of binder (B). Preferred amounts of binder (B) are from 0.1 to 5 percent by weight, more preferably 0.2 to 3 percent by weight of the (dry) weight of the material.

The coated material can comprise at least one pigment and/or at least one dyestuff. The amount of the at least one pigment and/or dyestuff is in general from 0.05 to 10 percent by weight, preferably 0.1 to 5 percent by weight, more preferably 0.2 to 3.5 percent by weight of the (dry) weight of the material.

The method of coating or treating the non-living material is not limited to a specific technology. Coating may be performed by dipping or submerging the non-living substrate into the formulation or by spraying the formulation onto the surface of the non-living material. After treating the treated non-living substrate may be dried simply at ambient temperatures.

Accordingly, no sophisticated technology is necessary for the coating, and therefore the coating process may be carried out by the end-user itself in at low-scale.

For instance, a typical end-user may coat/treat a net itself, e.g. within its household, using the formulation according to the present invention. For this purpose, it is in particular advantageous to use a kit as herein defined.

In an embodiment, the present invention provides a polymer, a fibre, a thread, a yarn, a net or weave comprising one or more 4-(trifluoromethyl)pyridine compounds (listed in Table 1), where also incorporated can be one or more other customary materials used to make such a polymer, and the polymer, a fibre, a thread, a yarn, a net or weave optionally can further incorporate one or more other insecticides and/or synergists.

In an embodiment, the present invention provides a net or weave incorporated with one or more 4-(trifluoromethyl)pyridine compounds (such as those pyridines listed in Table 1), which optionally further incorporates one or more other insecticides and/ or synergists.

As described in the art, 4-(trifluoromethyl)pyridine compounds useful in the methods and other aspects of the present invention can be used alone or in combination with another insecticide, synergist, insect repellent, chemosterilant, flame retardant, UV protector/ absorber, and/or additives for controlling release characteristics.

When used in accordance with the invention, a 4-(trifluoromethyl)pyridine compound of Table 1 may be used alone to control a fly or mosquito or used in combination with one or other known insecticides and/or one or more additives (such as synergists) - in polymers for making non-living substrates, such as nets and weaves, for formulations for treating non-living substrates, such as nets and weaves, in IRS products and space-spraying products.

In an embodiment, the present invention provides a composition (useful for coating a polymeric material or a product therefrom, or a useful as a spray product) comprising one or more pyridine compounds selected from the 4-(trifluoromethyl)pyridine compounds of Table 1, which optionally further comprises one or more other insecticide and/or synergists and one or more other additives.

Examples of synergists are piperonylbutoxide (PBO), sebacic esters, fatty acids, fatty acid esters, vegetable oils, esters of vegetable oils, alcohol alkoxylates and antioxidants.

Suitable sebacic esters are for example dimethyl sebacate, diethyl sebacate, dibutyl sebacate, dibenzyl sebacate, bis(N-succinimidyl)sebacate, bis(2-ethylhexyl)sebacate, bis(1-octyloxy-2,2,6,6-tetramethyl-4-piperidyl)sebacate, bis(2,2,6,6-tetramethyl-4-piperidyl)sebacate and bis(1,2,2,6,6-pentamethyl-4-piperidinyl)sebacate (BLS292).

Suitable fatty acids are (preferably mono- or polyunsaturated) fatty acids having a chain length of 12 to 24 carbon atoms, for example palmitoleic acid, oleic acid, elaidic acid, vaccenic acid, icosenic acid, cetoleic acid, erucic acid, nervonic acid, linoleic acid, alpha-linolenic acid, gamma-linolenic acid, arachidonic acid, timnodonic acid, clupanodonic acid and cervonic acid. Particular preference is given to oleic acid, linoleic acid, alpha-linolenic acid and gamma-linolenic acid.

Suitable fatty acid esters are preferably methyl or ethyl esters of the above-recited fatty acids. Methyl esters are particularly preferred. Fatty acids and their esters can each also be present in mixtures.

Useful vegetable oils include all plant-derivable oils customarily usable in agrochemical compositions. As examples there may be mentioned sunflower oil, rapeseed oil, olive oil, castor oil, colza oil, maize kernel oil, cottonseed oil and soybean oil. Rapeseed oil is preferred.

Suitable esters of vegetable oils are methyl or ethyl esters of the above-recited oils. Methyl esters are preferred.

Antioxidants useful as additives include for example butylhydroxytoluene, butylhydroxyanisole and L-ascorbic acid.

Plant essential oils may also be used in an indoor residual spray compositions; examples are those selected from citronella, peppermint oil, d-limonene and abies sibirica. These plant essential oil materials are known and used for other uses and can be prepared by a skilled artisan by employing known methods and also are available commercially.

In another embodiment, in the practice of the methods and other aspects of the invention, pyridines selected from the 4-(trifluoromethyl)pyridine compounds of Table 1 are useful in combination with other insecticides applied either simultaneously or sequentially. In particular, it has been found that mosquitoes which pick-up a 4-(trifluoromethyl)pyridine compound of Table 1 are knocked down or debilitated and thereby become more prone to being controlled by the combination of such 4-(trifluoromethyl)pyridines with other suitable insectides.

In addition to at least one defined active ingredient from the group of a 4-(trifluoromethyl)pyridine compound of Table 1, the methods, compositions, polymer, product, substrate and/or integrated mosquito management solution according to the invention may contain one or more further insecticidally active ingredients, whether simultaneously or sequentially. Particularly examples are one or more active ingredients from the class of organophosphates, pyrethroids, carbamates, methoxyacrylates, oxadiazines, neonicotinoids, pyrroles, bisamides and also DDT, chlorantraniliprole, cyantraniliprole, deltamethrin, lambda-cyhalothrin, pirimiphos-methyl, permethrin, indoxacarb, nicotine, bensultap, cartap, spinosad, camphechlor, chlordane, endosulfan, gamma-HCH, HCH, heptachlor, lindane, methoxychlor, acetoprole, ethiprole, fipronil, pyrafluprole, pyriprole, vaniliprole, avermectin, emamectin, emamectin-benzoate, ivermectin, milbemycin, diofenolan, epofenonane, fenoxycarb, hydroprene, kinoprene, methoprene, pyriproxifen, triprene, chromafenozide, halofenozide, methoxyfenozide, tebufenozide, bistrifluoron, chlofluazuron, diflubenzuron, fluazuron, flucycloxuron, flufenoxuron, hexaflumuron, lufenuron, novaluron, noviflumuron, penfluoron, teflubenzuron, triflumuron, buprofezin, cyromazine, diafenthiuron, azocyclotin, cyhexatin, fenbutatin-oxide, chlorfenapyr, binapacyrl, dinobuton, dinocap, DNOC, fenazaquin, fenpyroximate, pyrimidifen, pyridaben, tebufenpyrad, tolfenpyrad, hydramethylnon, dicofol, rotenone, acequinocyl, fluacrypyrim, *Bacillus thuringiensis* strains, spirodiclofen, spiromesifen, spirotetramat, 3-(2,5-dimethylphenyl)-8-methoxy-2-oxo-1-azaspiro[4.5]dec-3-en-4-yl ethyl carbonate (alias: carbonic acid, 3-(2,5-dimethylphenyl)-8-methoxy-2-oxo-1-azaspiro[4.5]dec-3-en-4-yl ethyl ester, CAS-Reg.-No.: 382608-10-8), amitraz, propargite, flubendiamide, chloranthraniliprol, thiocyclam hydrogen oxalate, thiosultap-sodium, azadirachtin, *Bacillus* spec., *Beauveria* spec., Codlemone, *Metarrhizium* spec., *Paecilomyces* spec., *Thuringiensin, Verticillium* spec., aluminium phosphide, methylbromide, sulfurylfluoride, cryolite, pymetrozine, clofentezine, etoxazole, hexythiazox, amidoflumet, benclothiaz, benzoximate, bifenazate, bromopropylate, buprofezin, chinomethionate, chlordimeform, chlorobenzilate, chloropicrin, clothiazoben, cycloprene, cyflumetofen, dicyclanil, fenoxacrim, fentrifanil, flubenzimine, flufenerim, flutenzin, gossyplure, hydramethylnone, japonilure, metoxadiazone, petroleum, piperonylbutoxide, potassium oleate, pyridalyl, sulfluramid, tetradifon, tetrasul, triarathene and verbutin.

In particular, suitable combinations with at least one 4-(trifluoromethyl)pyridine compound of Table 1 may be made with permethrin, chlorfenapyr, pirimiphos-methyl, indoxacarb, lambda-cyhalothrin, deltamethrin, cyantraniliprole and chlorantraniliprole.

In a further aspect, the present invention provides a method for protecting humans and mammals against blood feeding dipteran, triatominae or cimicidae insects (incl. mosquitoes), the method comprising applying to such blood feeding insect or to a locus of potential or known interaction between the human or mammal and such insect, a vector control solution comprising a knockdown or blood feed inhibiting effective amount of a compound selected from the group consisting of a 4-(trifluoromethyl)pyridine compound as defined in Table 1.

Another aspect of the invention is a method for controlling the spread of a vector-borne disease, comprising: identifying a mosquito vector; and contacting the mosquito vector or its environment with a vector control solution comprising a knockdown or blood feeding inhibiting effective amount of a compound selected from the group consisting of a 4-(trifluoromethyl)pyridine compound as defined in Table 1.

An aspect of the invention also includes a knockdown or blood feed inhibiting method which comprises contacting a mosquito or its environment with a a vector control solution comprising a knockdown or blood feed inhibiting effective amount of a compound selected from the group consisting of a 4-(trifluoromethyl)pyridine compound as defined in Table 1.

The present invention also provides a method, comprising: (i) identifying a locus of potential or known interaction between a target insect vector (such as a mosquito vector) and a mammal, including a human, susceptible to pathogenic disease infection when contacted by such vector and (ii) positioning a vector control solution at the locus, wherein the solution includes a knockdown or blood feed inhibiting effective amount of a compound selected from the group consisting of 4-(trifluoromethyl)pyridine as defined in Table 1.

The present inventon through control of mosquitos would also be expected to control the many viruses carried by such vectors. As an example, control of the mosquitos of the genus *Aedes* by use of one or more of the defined 4-(trifluoromethyl)pyridine compounds Table 1, as part of a vector control solution, may control the Zika infections. Examples of mosquitos reported to spread the Zika virus are the *Aedes* mosquitoes, such as *Aedes aegypti* and *Aedes albopictus.* Accordingly, in an aspect, the present invention provide a method of controlling Zika virus infection, wherein one or more of the defined compounds Table 1 is present in a knockdown or blood feed inhibiting effective amount in the vicinity of *Aedes* mosquitoes, such as *Aedes aegypti* and *Aedes albopictus.* In the vicinity of the mosquitoes is meant areas where mosquitos are likely to be present, such as in the environment in general, specifically in a room, or at the site of a mosquito biting an individual or mammal, for example, on the skin surface

In each of the methods according to present invention, the vector control solution is preferably one or more of a composition, a product and a treated article, each comprising a compound selected from the group consisting of a 4-(trifluoromethyl)pyridine compound as defined in Table 1.

A "fibre" as used in the present invention refers only to a fine, threadlike piece, generally made of natural material, such as cotton, or jute.

In each aspect and embodiment of the invention, "consisting essentially" and inflections thereof are a preferred embodiment of "comprising" and its inflections, and "consisting of" and inflections thereof are a preferred embodiment of "consisting essentially of" and its inflections.

In each aspect and embodiment of the invention, the terms "effective amount", "knockdown effective amount" and "blood feed inhibiting effective amount" in reference to the use of the 4-(trifluoromethyl)pyridine of Table 1 in such methods, products, compositions and integrated solutions, shall mean an amount of 4-(trifluoromethyl)pyridine of Table 1 that can be picked-up by the target insect resulting in knockdown or blood feed inhibition in a manner which provides suitable control of such insect.

The disclosure in the present application makes available each and every combination of embodiments disclosed herein.

The following Examples serve to illustrate the invention. They do not limit the invention.

### EXAMPLES

### PREPARATION EXAMPLES:

### Example P1 - Compound (1.5) - 5-[4-(trifluoromethyl)-3-pyridyl]-1,2,4-oxathiazol-3-one

4-Trifluoromethyl-nicotinamide (285 mg, 1.50 mmol) was suspended in toluene (5 ml), and chlorocarbonyl sulfenyl chloride (42.0 µl, 0.50 mmol) was added. The reaction mixture was stirred at reflux for 3 hours. Then the reaction mixture was filtered, and the filtrate was concentrated. The residue was purified by Flashmaster (medium column, eluent: CycHex/EtOAc) to give 67 mg of 5-[4-(trifluoromethyl)-3-pyridyl]-1,2,4-oxathiazol-3-one as a yellow solid.

### Example P2 - Compound (1.7) - 3-isopropyl-5-[4-(trifluoromethyl)-3-pyridyl]-1,3,4-oxadiazol-2-one

The compound (1.7) can be prepared as described for the analogous unsubstituted phenyl derivative in N. Matsumura, Y. Otsuji, E. Imoto, Nippon Kagaku Kaishi 1976, 5, 782-784.
To a solution of N'-isopropyl-4-(trifluoromethyl)pyridine-3-carbohydrazide (74 mg, 0.28 mmol) in pyridine (1 ml) ethylchloroformate (0.062 ml, 0.57 mmol) was added at room temperature. The reaction mixture was stirred 3 days at room temperature. A small portion of toluene was added and the solvent was removed under vacuum at 45°C. The residue was purified by Combiflash with cyclohexane and ethylacetate as eluents to give 15 mg of 3-isopropyl-5-[4-(trifluoromethyl)-3-pyridyl]-1,3,4-oxadiazol-2-one as a yellow oil.

### Example P3 - Compound (1.12) - (isopropylamino) 4-(trifluoromethyl)pyridine-3-carboxylate

The compound can be prepared as described for the analogous unsubstituted phenyl derivative in D. Geffken, Chem. Ber., 1986, 119(2), 744-746.
N-Isopropyl-hydroxylamine hydrochloride (1.84 g, 16.5 mmol) was suspended in dichloromethane (50 ml), and DBU(2.24 ml) and pyridine (2.41 ml) were added. The reaction mixture was cooled down to 0°C, and 4-(trifluoromethyl)pyridine-3-carbonyl chloride in 25 ml dichloromethane was added dropwise with a dropping funnel. The reaction mixture was stirred for 2 hours at 0°C and 2 days at room temperature. The reaction mixture was washed with a NaHCO3 solution, water and 1M HCl. The organic phase was dried over Na2SO4, filtered and concentrated. The residue was purified by Flashmaster with cyclohexane and ethylacetate as eluents to give 1.92 g of (isopropylamino) 4-(trifluoromethyl)pyridine-3-carboxylate as a yellow oil.

### BIOLOGY EXAMPLES:

### Examples B1- B10 Anopheles stephensi (Indian malaria mosquito)

The individual wells of six (6) well tissue culture plates were treated with 250 µl of an ethanol solution containing a test compound at a defined concentration. Once the deposits were dry, ten non-blood fed adult female *Anopheles stephensi* (between two to five day old) were added to each well, and sustained with a 10% sucrose solution in a cotton wool plug. Assessment of the knockdown after 1 hour (Tables B1- B10), and mortality after 24 and 48 hours (Tables B1 - B4) was carried out.

In case of multiple tests, the mean value is reported. Results are shown in Tables B1- B10.

### Examples B11 - B20: Tunnel Studies

The following tests are based on the "WHOPES tunnel tests" (described at http://apps.who.int/iris/bitstream/10665/80270/1/9789241505277 eng.pdf) For examples B11 - B15 the WHOPES tunnel test procedure was followed, except that live guniea pig baits were replaced with a Hemotek membrane feeding device, filled with blood and kept constant at 37 °C to mimic a human host.

For examples B11 - B14, the tunnel test was conductec with *Anopheles stephensi*;

For example B15, the tunnel test was conducted with Anopheles gambiae, resistant strain AKRON.

For examples B16 - B20, the WHOPES tunnel test procedure was followed with live guniea pig baits wherein B16 - B18 were conduced in Burkina Faso with Anopheles gambiae Kisumu, Anopheles coluzzii VK7 lab strain and Anopheles gambiae s.l. VK field strain, respectively; and B19 - B20 were conducted in Cote d'lvoire with Anopheles gambiae Kisumu and Anopheles gambiae Tiassale field strain, respectively.

The commercial standard Olyset nets used in certain examples are 2% w/w permethrin (approx. 1000 mg/m2, although not all of that is available on the surface at any one time, as a good proportion is in the polymer).

All tests were done under standardised laboratory controlled conditions (27 °C +2 °C under subdued light). The tunnel apparatus was a 60 x 30 x 30 cm glass case open at each end and the floor was lined with wet blue roll in order to maintain humidity. A 30 x 30 x 30 cm cage, covered in untreated netting, was placed at the release end. The test nets were cut to 25 x 25 cm squares and fitted into cardboard frames that allow a 20 x 20 cm area to be exposed. Nine holes, 1 cm in diameter, were made in the netting in the pattern specified in the WHOPES test. The frames were then fitted into the tunnel one third of the way down its length. A Hemotek device, filled with blood and kept constant at 37 °C to mimic a human host, was placed in the smaller area of the glass tunnel behind the test netting, and the end of the tunnel closed with a netting screen. A human sat at the Hemotek end of the tunnels for the duration of the test. At the start of the test, 100 female mosquitoes (non-blood fed, aged 5-8 days) were introduced into the cage. Mosquitoes are free to fly in the tunnel but have to make contact with the piece of netting and locate the holes in it before passing through to reach the bait.

After 7 hours the mosquitoes were counted in each section of the tunnel. The number of mosquitoes knocked down and blood fed were recorded. Blood-feeding inhibition was assessed by comparing the proportion of blood-fed females (alive or dead) in treated and control tests. Four tunnels were run simultaneously each day, with one control containing untreated netting included with all runs. Three replicates were carried out per treatment. In case of multiple tests, the mean value is reported. Results are shown in Tables B11 - B20.

### Examples B21 and B22: Bottle Assay

Based on the "CDC bottle assay" (described at http://www.cdc.gov/malaria/resources/pdf/fsp/ir_manual/ir_cdc_bioassay_en.pdf) 1 ml of ethanol containing a test compound at a defined concentration was added to a 250 ml glass bottle and the bottles were placed on a rolling table to coat the inner surfaces as the solvent evaporated. Once dry, twenty five non-blood fed adult female mosquitoes of the appropriate species and strains (each three day old) were aspirated from the stock culture and gently blown into the exposure bottles. The lid of the bottle was replaced and the bottle placed upright out of direct sun light under standard culture conditions, nominally 28 °C and 60 - 80% relative humidity.

A stopwatch was started, and the assessment of the knock-down were made after 15 mins and 60 minutes. A mosquito was said to be knocked down if it was unable to stand, following the CDC definition. The bottles were replaced in an upright position when not being assessed.

After one hour the mosquitoes were carefully removed from the bottle with an aspirator and placed in a recovery cup. The mosquitoes were supplied with a 10% sucrose solution on a cotton wool bung, and stored under culture conditions. Assessments of the mortality were made after 24 hours and 48 hours (B21).

Each treatment was replicated a minimum of four times, with the mean knockdown or mortality recorded. In each study, a set of bottles was infested with a known insecticide susceptible strain of mosquitoes from the same genera as the resistant strains. Results are shown in Tables B21 and B22.

### Examples B23: Aedes aegypti and Anopheles stephensi (Indian malaria mosquito)

The individual wells of a twelve (12) well tissue culture plates were treated with 100 µl of an ethanol solution containing a test compound at a defined concentration. Once the deposits were dry, five non-blood fed adult female Aedes aegypti or Anopheles stephensi (beween two to five day old) were added to each well, and sustained with a 10% sucrose solution in a cotton wool plug. Assessment of the knockdown after 1 hour was carried out. Where multiple replicates of a treatment have been undertaken, the mean of those replicates is reported. Results are shown in Tables B23.

**Table B1:6 well plate w/10 mosq**

| | | KD | - | 24 hr mort | - | 48 hr mort | - |
|---|---|---|---|---|---|---|---|
| | - Rate (ppm) | - | + Comp. (1.3) | - | + Comp. (1.3) | - | + Comp. (1.3) |
| Permethrin | 2 | 13.3 | 66.7 | 66.7 | 100.0 | 80.0 | 100.0 |
| | 1 | 10.0 | 33.3 | 40.0 | 73.3 | 46.7 | 76.7 |
| | 0.5 | 0.0 | 33.3 | 23.3 | 60.0 | 30.0 | 60.0 |
| | 0.25 | 0.0 | 23.3 | 16.7 | 36.7 | 20.0 | 40.0 |
| | 0.125 | 0.0 | 13.3 | 3.3 | 16.7 | 0.0 | 20.0 |
| - | 0.0625 | 0.0 | 13.3 | 0.0 | 6.7 | 0.0 | 6.7 |
| chlorfenapyr | 2 | 0.0 | 26.7 | 40.0 | 63.3 | 90.0 | 93.3 |
| | 1 | 0.0 | 16.7 | 6.7 | 33.3 | 50.0 | 60.0 |
| | 0.5 | 0.0 | 13.3 | 0.0 | 10.0 | 16.7 | 23.3 |
| | 0.25 | 0.0 | 10.0 | 0.0 | 0.0 | 3.3 | 3.3 |
| | 0.125 | 0.0 | 6.7 | 0.0 | 0.0 | 0.0 | 0.0 |
| - | 0.0625 | 0.0 | 6.7 | 0.0 | 0.0 | 0.0 | 0.0 |
| Comp. (1.3) | 0.1 | | 23.3 | 0.0 | | 0.0 | |

**Table B2:**

| | | KD | - | 24 hr mort | - | 48 hr mort | |
|---|---|---|---|---|---|---|---|
| | Rate (ppm) | | + Comp. (1.3) | | + Comp. (1.3) | | + Comp. (1.3) |
| Permethrin | 2 | 0.0 | 100.0 | 80.0 | 100.0 | 80.0 | 100.0 |
| | 1 | 0.0 | 100.0 | 40.0 | 100.0 | 70.0 | 100.0 |
| | 0.5 | 0.0 | 100.0 | 30.0 | 100.0 | 50.0 | 100.0 |
| | 0.25 | 0.0 | 100.0 | 10.0 | 100.0 | 10.0 | 90.0 |
| | 0.125 | 0.0 | 100.0 | 0.0 | 100.0 | 0.0 | 80.0 |
| | 0.0625 | 0.0 | 100.0 | 0.0 | 100.0 | 0.0 | 70.0 |
| Chlorfenapyr | 2 | 0.0 | 100.0 | 50.0 | 100.0 | 100.0 | 100.0 |
| | 1 | 0.0 | 100.0 | 20.0 | 95.0 | 40.0 | 90.0 |
| | 0.5 | 0.0 | 100.0 | 0.0 | 85.0 | 0.0 | 55.0 |
| | 0.25 | 0.0 | 100.0 | 0.0 | 80.0 | 0.0 | 40.0 |
| | 0.125 | 0.0 | 100.0 | 0.0 | 85.0 | 0.0 | 15.0 |
| | 0.0625 | 0.0 | 100.0 | 0.0 | 80.0 | 0.0 | 10.0 |
| Comp. (1.3) | 0.5 | | 60.0 | | 7.0 | | 10.0 |

**Table B3:**

| | | KD | - | 24 hr mort | - | 48 hr mort | - |
|---|---|---|---|---|---|---|---|
| | Rate (ppm) | | + Comp. (1.3) | | + Comp. (1.3) | | + Comp. (1.3) |
| pirimiphos-methyl | 1 | 10.0 | 100.0 | 100.0 | 100.0 | 100.0 | 100.0 |
| | 0.5 | 0.0 | 100.0 | 100.0 | 100.0 | 100.0 | 100.0 |
| | 0.25 | 0.0 | 90.0 | 50.0 | 100.0 | 50.0 | 100.0 |
| | 0.125 | 0.0 | 90.0 | 0.0 | 60.0 | 0.0 | 50.0 |
| | 0.0625 | 0.0 | 100.0 | 0.0 | 0.0 | 0.0 | 10.0 |
| | 0.03125 | 0.0 | 80.0 | 0.0 | 0.0 | 0.0 | 0.0 |
| Comp. (1.3) | 0.5 | - | 73.0 | - | 0.0 | - | 0.0 |
| Ethanol | - | 0.0 | - | 0.0 | - | 0.0 | - |

**Table B4:**

| | - | KD | - | 24 hr mort | - | 48 hr mort | - |
|---|---|---|---|---|---|---|---|
| | Rate (ppm) | - | + Comp. (1.3) | | + Comp. (1.3) | - | + Comp. (1.3) |
| Indoxacarb | 100 | 0.0 | 30.0 | 0.0 | 20.0 | 0.0 | 10.0 |
| | 50 | 0.0 | 5.0 | 0.0 | 0.0 | 0.0 | 0.0 |
| | 25 | 0.0 | 5.0 | 0.0 | 0.0 | 0.0 | 0.0 |
| | 12.5 | 0.0 | 0.0 | 0.0 | 0.0 | 0.0 | 0.0 |
| Comp. (1.3) | 0.5 | | 17.5 | - | 2.5 | - | 2.5 |
| Ethanol | - | 0.0 | - | 0.0 | - | 0.0 | - |

**Table B5: Lambda**

| Treatement | Rate/ppm | Alone | Plus Compound (1.3) |
|---|---|---|---|
| Lambda-cyhalothrin | 0.2 | 40 | 100 |
| | 0.1 | 0 | 60 |
| | 0.05 | 0 | 20 |
| | 0.025 | 0 | 0 |

**Table B6: Deltamethrin**

| Treatment | Rate (ppm) | Alone | Plus Compound (1.3) |
|---|---|---|---|
| Deltamethrin | 1 | 100 | 100 |
| | 0.5 | 100 | 100 |
| | 0.25 | 100 | 100 |
| | 0.125 | 40 | 80 |
| | 0.0625 | 40 | 80 |
| | 0.03125 | 20 | 80 |

**Table B7: Indoxacarb**

| Treatement | Rate (ppm) | Alone | Plus Compound (1.3) |
|---|---|---|---|
| Indoxacarb | 200 | 0 | 40 |
| | 100 | 0 | 60 |
| | 50 | 0 | 40 |
| | 25 | 0 | 0 |

**Table B8: Cyantraniliprole**

| Treatment | Rate (ppm) | Alone | Plus Compound (1.3) |
|---|---|---|---|
| Cyantraniliprole | 200 | 20 | 100 |
| | 100 | 0 | 60 |
| | 50 | 0 | 60 |
| | 25 | 0 | 40 |
| | 12.5 | 0 | 40 |
| | 6.25 | 0 | 0 |

**Table B9: Chlorantraniliprole**

| Treatment | Rate (ppm) | Alone | Plus Compound (1.3) |
|---|---|---|---|
| Chlorantraniliprole | 200 | 40 | 80 |
| | 100 | 20 | 80 |
| | 50 | 0 | 60 |
| | 25 | 0 | 60 |
| | 12.5 | 0 | 0 |

**Table B10: Pirimiphos-methyl**

| Treatment | Rate (ppm) | Alone | Plus Compound (1.3) |
|---|---|---|---|
| Pirimiphos-methyl | 1 | 100.0 | 100.0 |
| | 0.5 | 100.0 | 100.0 |
| | 0.25 | 50.0 | 100.0 |
| | 0.125 | 0.0 | 60.0 |
| | 0.0625 | 0.0 | 0.0 |
| Compound (1.3) | 0.5 | | 0.0 |
| Ethanol | | 0.0 | |

**Table B11:**

| | | |
|---|---|---|
| Treatment | 24 hours | |
| Rate 10mg/m2 | % Mortality | % Blood fed |
| Permethrin | 24 | 31 |
| Compound (1.3) | 68 | 3 |
| Permethrin +compound (1.3) | 100 | 0 |

**Table B12:**

| Treatment | % Through net | % 1 hour KD | % Blood fed |
|---|---|---|---|
| Deltamethrin 5mg/m2 | 0.93 | 0 | 0 |
| Deltamethrin + compound (1.3) | 5 | 57 | 0 |
| Compound (1.3) 10mg/m2 | 9.28 | 41.24 | 3.09 |
| Control | 11 | 0 | 10 |

**Table B13:**

| Treatment | % Through net | % 24 hour mortality | % Blood fed |
|---|---|---|---|
| Deltamethrin 5mg/m2 | 67.6 | 12.96 | 52.77 |
| Deltamethrin + comound (1.3) | 9 | 91 | 5 |
| Compound (1.3) 10mg/m2 | 22.68 | 72.16 | 18.56 |
| Control | 72 | 5 | 67 |

**Table B14:**

| Treatement | % 24 hour mortality | % Blood fed |
|---|---|---|
| Indoxacarb 20mg/m2 | 7.6 | 31.4 |
| Compound (1.3) 10mg/m2 | 68 | 2.9 |
| Indoxacarb + compound (1.3) | 73.7 | 4.2 |

**Table B15:**

| Treatment | % blood fed |
|---|---|
| Untreated Net | 30.5 |
| Olyset | 13.7 |
| Compound (1.3) 25mg/m2 | 0.3 |
| Indoxacarb 200mg/m2 | 4.4 |
| Compound (1.3) 25mg/m2 + Indoxacarb 200mg/m2 | 2.9 |

**Table B16:**

| Treatment | Total no. mosq. | Blood fed | % Blood fed |
|---|---|---|---|
| Compound (1.3) 50 mg/m2 | 329 | 5 | 1.5 |
| Compound (1.3) 200 mg/m2 | 310 | 1 | 0.3 |
| Olyset | 282 | 1 | 0.4 |
| Untreated | 314 | 108 | 34.4 |

**Table B17:**

| Treatment | Total no. mosq. | Blood fed | % Blood fed |
|---|---|---|---|
| Compound (1.3) 50 mg/m2 | 300 | 10 | 3.3 |
| Compound (1.3) 200 mg/m2 | 404 | 6 | 1.5 |
| Olyset | 319 | 61 | 19.1 |
| Untreated | 390 | 150 | 38.5 |

**Table B18:**

| Treatment | Total no. mosq. | Blood fed | % Blood fed |
|---|---|---|---|
| Compound (1.3) 50 mg/m2 | 320 | 4 | 1.3 |
| Compound (1.3) 200 mg/m2 | 332 | 6 | 1.8 |
| Olyset | 268 | 34 | 12.7 |
| Untreated | 244 | 93 | 38.1 |

**Table B19:**

| Treatment | Total | Blood fed | % Blood fed |
|---|---|---|---|
| Compound (1.3) 50 mg/m2 | 280 | 70 | 25.0 |
| Compound (1.3) 200 mg/m2 | 320 | 74 | 23.1 |
| Olyset | 98 | 2 | 2.0 |
| Control | 319 | 281 | 88.1 |

**Table B20:**

| Treatment | Total | Blood fed | % Blood fed |
|---|---|---|---|
| Compound (1.3) 50 mg/m2 | 269 | 66 | 24.5 |
| Compound (1.3) 200 mg/m2 | 304 | 10 | 3.3 |
| Olyset | 99 | 24 | 24.2 |
| Control | 191 | 78 | 40.8 |

**Table B21:**

| Treatment | % 1 hour KD | 24 hour % mort | 48 hour % mort |
|---|---|---|---|
| Permethrin | 100 | 0 | 0 |
| Compound (1.3) | 86.66 | 0 | 0 |
| Flonicamid | 0 | 0 | 0 |
| Permethrin + flonicamid | 100 | 0 | 0 |
| Permethrin + compound (1.3) | 100 | 60 | 46.66 |
| Flonicamid + compound (1.3) | 80 | 0 | 0 |

**Table B22:**

| Treatment | % 1 hour KD | % 24 hour mortality |
|---|---|---|
| Compound (1.3) 10ug/bottle | 100 | 0 |
| Permethrin 1ug/bottle | 100 | 0 |
| PBO 400ug/bottle | 0 | 6.66 |
| Silicon oil 50ug/bottle | 0 | 0 |
| Compound (1.3) + permethrin | 100 | 80 |
| Compound (1.3) + PBO | 100 | 80 |
| Permethrin + PBO | 100 | 100 |
| Silicon oil + compound (1.3) | 100 | 18.75 |
| Silicon oil + permethrin | 73.33 | 0 |

**Table B23**

| | | *Aedes aegypti* | *Anopheles stephensi* |
|---|---|---|---|
| | Rate | | |
| Compound | PPM | 1 hour %KD | 1 hour %KD |
| 1.1 | 200 | 100 | 100 |
| | 20 | 100 | 100 |
| | 2 | 100 | 100 |
| | 0.2 | 100 | 40 |
| 1.2 | 200 | 100 | 100 |
| | 20 | 100 | 80 |
| | 2 | 100 | 60 |
| | 0.2 | 100 | 0 |
| 1.3 | 200 | 100 | 100 |
| | 20 | 100 | 100 |
| | 2 | 100 | 20 |
| | 0.2 | 100 | 20 |
| 1.4 | 200 | 100 | 100 |
| | 20 | 100 | 100 |
| | 2 | 100 | 100 |
| | 0.2 | 100 | 40 |
| 1.5 | 200 | 100 | 100 |
| | 20 | 100 | 100 |
| | 2 | 100 | 100 |
| | 0.2 | 100 | 60 |
| 1.6 | 200 | 100 | 40 |
| | 20 | 100 | 40 |
| | 2 | 100 | 20 |
| | 0.2 | 100 | 0 |
| 1.7 | 200 | 100 | 20 |
| | 20 | 100 | 0 |
| | 2 | 100 | 0 |
| | 0.2 | 100 | 0 |
| 1.8 | 200 | 100 | 100 |
| | 20 | 100 | 100 |
| | 2 | 100 | 100 |
| | 0.2 | 100 | 60 |
| 1.9 | 200 | 100 | 100 |
| | 20 | 100 | 100 |
| | 2 | 100 | 100 |
| | 0.2 | 100 | 20 |
| 1.10 | 200 | 100 | 100 |
| | 20 | 100 | 100 |
| | 2 | 100 | 60 |
| | 0.2 | 100 | 40 |
| 1.11 | 200 | 100 | Not tested |
| | 20 | 100 | Not tested |
| | 2 | 100 | Not tested |
| | 0.2 | 100 | Not tested |
| 1.12 | 200 | 100 | 100 |
| | 20 | 100 | 100 |
| | 2 | 100 | 80 |
| | 0.2 | 100 | 0 |
| 1.13 | 200 | 100 | 100 |
| | 20 | 100 | 100 |
| | 2 | 100 | 60 |
| | 0.2 | 0 | 0 |
| 1.14 | 200 | 100 | 100 |
| | 20 | 100 | 100 |
| | 2 | 100 | 40 |
| | 0.2 | 0 | 40 |
| 1.15 | 200 | 100 | 0 |
| | 20 | 100 | 0 |
| | 2 | 100 | 0 |
| | 0.2 | 100 | 0 |
| 1.16 | 200 | 100 | 0 |
| | 20 | 60 | 0 |
| | 2 | 60 | 0 |
| | 0.2 | 0 | 0 |
| 1.17 | 200 | 0 | 100 |
| | 20 | 0 | 20 |
| | 2 | 0 | 0 |
| | 0.2 | 0 | 0 |
| 1.18 | 200 | 100 | 40 |
| | 20 | 100 | 0 |
| | 2 | 100 | 0 |
| | 0.2 | 40 | 0 |
| 1.19 | 200 | 100 | 100 |
| | 20 | 100 | 100 |
| | 2 | 100 | 60 |
| | 0.2 | 0 | 40 |
| 1.20 | 200 | 100 | 100 |
| | 20 | 100 | 100 |
| | 2 | 40 | 60 |
| | 0.2 | 0 | 20 |
| 1.21 | 200 | 100 | 100 |
| | 20 | 0 | 100 |
| | 2 | 0 | 60 |
| | 0.2 | 0 | 20 |
| 1.22 | 200 | 100 | 100 |
| | 20 | 100 | 60 |
| | 2 | 100 | 60 |
| | 0.2 | 100 | 40 |
| 1.23 | 200 | 100 | 100 |
| | 20 | 100 | 40 |
| | 2 | 100 | 20 |
| | 0.2 | 40 | 0 |
| 1.24 | 200 | 100 | 100 |
| | 20 | 100 | 100 |
| | 2 | 100 | 60 |
| | 0.2 | 100 | 40 |
| 1.25 | 200 | 100 | 100 |
| | 20 | 100 | 100 |
| | 2 | 100 | 80 |
| | 0.2 | 100 | 80 |
| 1.26 | 200 | 100 | 100 |
| | 20 | 100 | 100 |
| | 2 | 100 | 0 |
| | 0.2 | 0 | 0 |
| 1.27 | 200 | 100 | 100 |
| | 20 | 100 | 100 |
| | 2 | 100 | 0 |
| | 0.2 | 0 | 0 |
| 1.28 | 200 | 100 | 100 |
| | 20 | 60 | 40 |
| | 2 | 40 | 0 |
| | 0.2 | 20 | 0 |
| 1.29 | 200 | 100 | 100 |
| | 20 | 0 | 100 |
| | 2 | 0 | 60 |
| | 0.2 | 0 | 20 |

## Claims

1. A method for controlling a nuisance or disease carrying or haematophagous dipteran, triatominae or cimicidae insect pest selected from flies, bed bugs and kissing bugs comprising: applying a composition containing a knockdown or blood feed inhibiting effective amount of a 4-(trifluoromethyl)pyridine compound to such insect pest or to a locus where such control is desired, wherein the 4-(trifluoromethyl)pyridine compound is selected from the group consisting of a compound represented by the formulae 1.1 - 1.29.
| | | | | | |
|---|---|---|---|---|---|
| 1.1 | 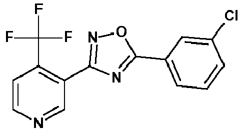 | 1.2 | 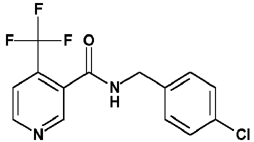 | 1.3 | 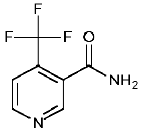 |
| 1.4 | 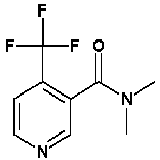 | 1.5 | 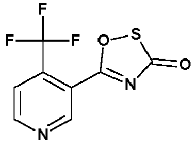 | 1.6 | 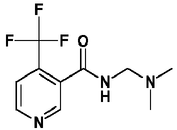 |
| 1.7 | 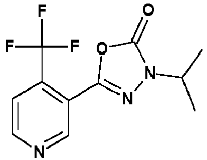 | 1.8 | 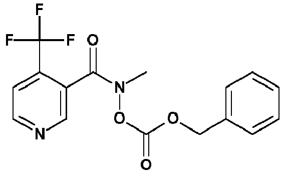 | 1.9 | 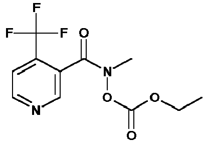 |
| 1.10 | 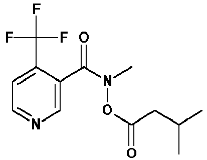 | 1.11 | 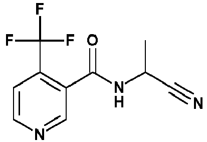 | 1.12 | 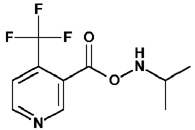 |
| 1.13 | 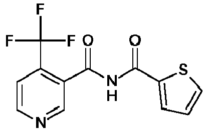 | 1.14 | 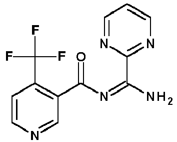 | 1.15 | 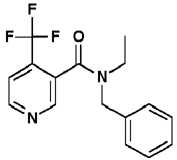 |
| 1.16 | 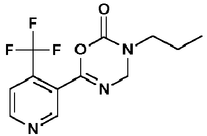 | 1.17 | 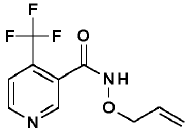 | 1.18 | 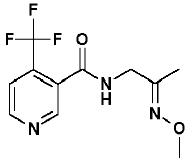 |
| 1.19 | 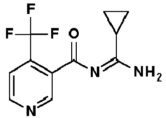 | 1.20 | 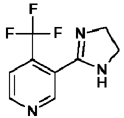 | 1.21 | 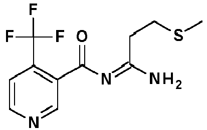 |
| 1.22 | 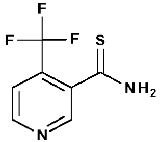 | 1.23 | 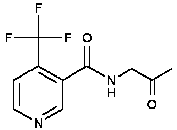 | 1.24 | 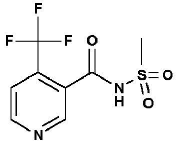 |
| 1.25 | 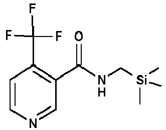 | 1.26 | 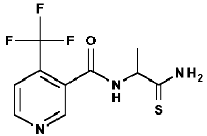 | 1.27 | 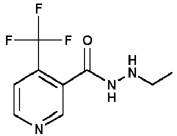 |
| 1.28 | 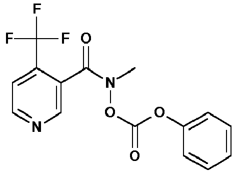 | 1.29 | 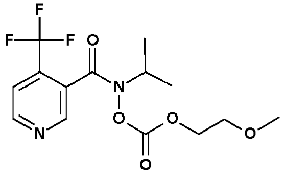 | | |

2. The method of claim 1, wherein said dipteran insect pest is a fly.

3. The method of claim 1, wherein said cimicidae insect pest is a bed bug.

4. The method of claim 1, wherein said triatominae insect pest is a kissing bug.

5. A method for controlling insect pest selected from flies, bed bugs and kissing bugs, the method comprising applying to the insect pest or to a locus of potential or known interaction between the human or mammal and the insect pest, an active compound composition comprising a knockdown or blood feed inhibiting effective amount of a composition comprising a compound selected from the group consisting of a pyridine compound of formulae (1.1) - (1.29) as described in claim 1.

6. The method of claim 5, wherein the active compound composition is applied to a non-living material or substrate at a locus of potential or known interaction between the insect pest and said human or mammal.

7. A method for controlling an insect pest selected from flies, bed bugs and kissing bugs by knockdown or blood feed inhibition which method comprises (a) applying a knockdown or blood feed inhibiting effective amount of a liquid composition comprising at least one 4-(trifluoromethyl)pyridine compound selected from a compound of formulae 1.1 - 1.29 as defined in claim 1, and a polymeric binder, and optionally, one or more other insecticides, and/or synergists, to a surface of a dwelling; and/or (b) placing a substrate or non-living material incorporated with said at least one said 4-(trifluoromethyl)pyridine compound, and optionally an additive, one or more other insecticides, and/or synergists, within a dwelling.

8. The method of claims 1 - 7, wherein the 4-(trifluoromethyl)pyridine compound is selected from a compound represented by the formulae 1.3.

9. The method of claims 1 - 8, wherein said composition further comprises at least one insecticide selected from permethrin, chlorfenapyr, pirimiphos-methyl, indoxacarb, lambda-cyhalothrin, deltamethrin, cyantraniliprole and chlorantraniliprole.
